# EUROPEAN PATENT APPLICATION

(11) **EP 1 260 229 A2**
(43) Date of publication of application: **27.11.2002**
(21) Application number: 02011048.2
(22) Date of filing: 17.05.2002
(51) Int. Cl.: A61K 38/25, G01N 33/50, A61P 19/02, A61P 21/00, A61P 37/00, A61K 39/395, A61K 31/7088, A61K 48/00

(54) **Anti-autoimmune composition comprising inhibitors of growth hormone-releasing factor activity**

(30) Priority: 18.05.2001 JP 2001148607
(71) Applicant: Sumitomo Pharmaceuticals Company, Limited, Osaka 541-8510 (JP); Sumitomo Chemical Company, Limited, Osaka-shi, Osaka-fu (JP)
(72) Inventor: Ikushima, Hideto, Sakai-shi, Osaka-fu (JP)
(74) Representative: VOSSIUS & PARTNER

(57) **Abstract**

A novel anti-autoimmune composition and a method of screening for the active ingredient of an anti-autoimmune composition are provided. The anti-autoimmune composition of the invention is characterized by its comprising a substance having GRF inhibitory activity as an active ingredient. The screening method of the invention is a method of screening investigational substances for a substance having GRF inhibitory activity with the inhibition of the GRF receptor-mediated action of GRF being used as a marker, by which method the active ingredient of an anti-autoimmune composition can be acquired.

SEQUENCE LISTING

<110> Sumitomo Pharmaceuticals Company, Limited
<120> Novel anti autoimmune composition by inhibition of GRF action
<130> 20102JP
<150> JP2001-148607
<151> 2001-5-18
<160> 8
<170> PatentIn Ver. 2.1

<210> 1
<211> 327
<212> DNA
<213> Homo sapiens
<400> 1
atgccactct gggtgttctt ctttgtgatc ctcaccctca gcaacagctc ccactgctcc 60
ccacctcccc ctttgaccct caggatgcgg cggtatgcag atgccatctt caccaacagc 120
taccggaagg tgctgggcca gctgtccgcc cgcaagctgc tccaggacat catgagcagg 180
cagcagggag agagcaacca agagcgagga gcaagggcac ggcttggtcg tcaggtagac 240
agcatgtggg cagaacaaaa gcaaatggaa ttggagagca tcctggtggc cctgctgcag 300
aagcacagca ggaactccca gggatga 327
<210> 2
<211> 108
<212> PRT
<213> Homo sapiens
<400> 2
Met Pro Leu Trp Val Phe Phe Phe Val Ile Leu Thr Leu Ser Asn Ser
1               5                   10                  15
Ser His Cys Ser Pro Pro Pro Pro Leu Thr Leu Arg Met Arg Arg Tyr
            20                  25                  30

Ala Asp Ala Ile Phe Thr Asn Ser Tyr Arg Lys Val Leu Gly Gln Leu
        35                  40                  45
Ser Ala Arg Lys Leu Leu Gln Asp Ile Met Ser Arg Gln Gln Gly Glu
    50                  55                  60
Ser Asn Gln Glu Arg Gly Ala Arg Ala Arg Leu Gly Arg Gln Val Asp
65                  70                  75                  80
Ser Met Trp Ala Glu Gln Lys Gln Met Glu Leu Glu Ser Ile Leu Val
                85                  90                  95
Ala Leu Leu Gln Lys His Ser Arg Asn Ser Gln Gly
            100                 105
<210> 3
<211> 1617
<212> DNA
<213> Homo sapiens
<400> 3
agcagccaag gcttactgag gctggtggag ggagccactg ctgggctcac catggaccgc 60
cggatgtggg gggcccacgt cttctgcgtg ttgagcccgt taccgaccgt attgggccac 120
atgcacccag aatgtgactt catcacccag ctgagagagg atgagagtgc ctgtctacaa 180
gcagcagagg agatgcccaa caccaccctg ggctgccctg cgacctggga tgggctgctg 240
tgctggccaa cggcaggctc tggcgagtgg gtcaccctcc cctgcccgga tttcttctct 300
cacttcagct cagagtcagg ggctgtgaaa cgggattgta ctatcactgg ctggtctgag 360
ccctttccac cttaccctgt ggcctgccct gtgcctctgg agctgctggc tgaggaggaa 420
tcttacttct ccacagtgaa gattatctac accgtgggcc atagcatctc tattgtagcc 480
ctcttcgtgg ccatcaccat cctggttgct ctcaggaggc tccactgccc ccggaactac 540
gtccacaccc agctgttcac cacttttatc ctcaaggcgg gacgtgtgtt cctgaaggat 600
gctgcccttt tccacagcga cgacactgac cactgcagct tctccactgt tctatgcaag 660
gtctctgtgg ccgcctccca tttcgccacc atgaccaact tcagctggct gttggcagaa 720
gccgtctacc tgaactgcct cctggcctcc acctccccca gctcaaggag agccttctgg 780
tggctggttc tcgctggctg ggggctgccc gtgctcttca ctggcacgtg ggtgagctgc 840
aaactggcct tcgaggacat cgcgtgctgg gacctggacg acacctcccc ctactggtgg 900

atcatcaaag ggcccattgt cctctcggtc ggggtgaact ttgggctttt tctcaatatt 960
atccgcatcc tggtgaggaa actggagcca gctcagggca gcctccatac ccagtctcag 1020
tattggcgtc tctccaagtc gacacttttc ctgatcccac tctttggaat tcactacatc 1080
atcttcaact tcctgccaga caatgctggc ctgggcatcc gcctccccct ggagctggga 1140
ctgggttcct tccagggctt cattgttgcc atcctctact gcttcctcaa ccaagaggtg 1200
aggactgaga tctcacggaa gtggcatggc catgaccctg agcttctgcc agcctggagg 1260
acccgtgcta agtggaccac gccttcccgc tcggcggcaa aggtgctgac atctatgtgc 1320
taggctgcct catcacgcca ctggagtcca cacttgaatt tgggcagcta ccacgggtct 1380
gccatgctct ggaggagcaa gggggccaca tccccacccc agctgttacc cagcccgggg 1440
caggtgcagc ccttcctccc tgtctctgca tctgactctc ttttgaggtc cctgtatgtc 1500
tacctctgac ttctgtggtc cctctgtgtc tgctctcatc cattcctctt actggggcct 1560
ggggctctag cccaaggctc agaggagcca ataaacctgt aaatgaaaaa aaaaaaa 1617
<210> 4
<211> 423
<212> PRT
<213> Homo sapiens
<400> 4
Met Asp Arg Arg Met Trp Gly Ala His Val Phe Cys Val Leu Ser Pro
1               5                   10                  15
Leu Pro Thr Val Leu Gly His Met His Pro Glu Cys Asp Phe Ile Thr
            20                  25                  30
Gln Leu Arg Glu Asp Glu Ser Ala Cys Leu Gln Ala Ala Glu Glu Met
        35                  40                  45
Pro Asn Thr Thr Leu Gly Cys Pro Ala Thr Trp Asp Gly Leu Leu Cys
    50                  55                  60
Trp Pro Thr Ala Gly Ser Gly Glu Trp Val Thr Leu Pro Cys Pro Asp
65                  70                  75                  80
Phe Phe Ser His Phe Ser Ser Glu Ser Gly Ala Val Lys Arg Asp Cys
                85                  90                  95
Thr Ile Thr Gly Trp Ser Glu Pro Phe Pro Pro Tyr Pro Val Ala Cys

            100                 105                 110
Pro Val Pro Leu Glu Leu Leu Ala Glu Glu Glu Ser Tyr Phe Ser Thr
        115                 120                 125
Val Lys Ile Ile Tyr Thr Val Gly His Ser Ile Ser Ile Val Ala Leu
    130                 135                 140
Phe Val Ala Ile Thr Ile Leu Val Ala Leu Arg Arg Leu His Cys Pro
145                 150                 155                 160
Arg Asn Tyr Val His Thr Gln Leu Phe Thr Thr Phe Ile Leu Lys Ala
                165                 170                 175
Gly Arg Val Phe Leu Lys Asp Ala Ala Leu Phe His Ser Asp Asp Thr
            180                 185                 190
Asp His Cys Ser Phe Ser Thr Val Leu Cys Lys Val Ser Val Ala Ala
        195                 200                 205
Ser His Phe Ala Thr Met Thr Asn Phe Ser Trp Leu Leu Ala Glu Ala
    210                 215                 220
Val Tyr Leu Asn Cys Leu Leu Ala Ser Thr Ser Pro Ser Ser Arg Arg
225                 230                 235                 240
Ala Phe Trp Trp Leu Val Leu Ala Gly Trp Gly Leu Pro Val Leu Phe
                245                 250                 255
Thr Gly Thr Trp Val Ser Cys Lys Leu Ala Phe Glu Asp Ile Ala Cys
            260                 265                 270
Trp Asp Leu Asp Asp Thr Ser Pro Tyr Trp Trp Ile Ile Lys Gly Pro
        275                 280                 285
Ile Val Leu Ser Val Gly Val Asn Phe Gly Leu Phe Leu Asn Ile Ile
    290                 295                 300
Arg Ile Leu Val Arg Lys Leu Glu Pro Ala Gln Gly Ser Leu His Thr
305                 310                 315                 320
Gln Ser Gln Tyr Trp Arg Leu Ser Lys Ser Thr Leu Phe Leu Ile Pro
                325                 330                 335
Leu Phe Gly Ile His Tyr Ile Ile Phe Asn Phe Leu Pro Asp Asn Ala

            340                 345                 350
Gly Leu Gly Ile Arg Leu Pro Leu Glu Leu Gly Leu Gly Ser Phe Gln
        355                 360                 365
Gly Phe Ile Val Ala Ile Leu Tyr Cys Phe Leu Asn Gln Glu Val Arg
    370                 375                 380
Thr Glu Ile Ser Arg Lys Trp His Gly His Asp Pro Glu Leu Leu Pro
385                 390                 395                 400
Ala Trp Arg Thr Arg Ala Lys Trp Thr Thr Pro Ser Arg Ser Ala Ala
                405                 410                 415
Lys Val Leu Thr Ser Met Cys
            420
<210> 5
<211> 10
<212> PRT
<213> Artificial Sequence
<400> 5
Glu Gln Lys Lue Ile Ser Glu Glu Asp Ile
5 10
<210> 6
<211> 6
<212> PRT
<213> Artificial Sequence
<400> 6
His His His His His His
                5
<210> 7
<211> 7
<212> PRT
<213> Artificial Sequence
<400> 7

340 345 350
Gly Leu Gly Ile Arg Leu Pro Leu Glu Leu Gly Leu Gly Ser Phe Gln
355 360 365
Gly Phe Ile Val Ala Ile Leu Tyr Cys Phe Leu Asn Gln Glu Val Arg
370 375 380
Thr Glu Ile Ser Arg Lys Trp His Gly His Asp Pro Glu Leu Leu Pro
385 390 395 400
Ala Trp Arg Thr Arg Ala Lys Trp Thr Thr Pro Ser Arg Ser Ala Ala
405 410 415
Lys Val Leu Thr Ser Met Cys
420
<210> 5
<211> 10
<212> PRT
<213> Artificial Sequence
<400> 5
Glu Gln Lys Lue Ile Ser Glu Glu Asp Ile
5 10
<210> 6
<211> 6
<212> PRT
<213> Artificial Sequence
<400> 6
His His His His His His
                5
<210> 7
<211> 7
<212> PRT
<213> Artificial Sequence
<400> 7

## Description

### TECHNICAL FIELD

The present invention relates to an anti-autoimmune composition, that is a prophylactic or therapeutic drug for autoimmune diseases. The present invention further relates to a method of screening for a novel active ingredient for such an anti-autoimmune composition.

### BACKGROUND ART

Rheumatoid arthritis, multiple sclerosis and systemic lupus erythematosus, among other diseases, present with apparently divergent pathologic signs but all are diseases involving inflammations at affected sites. These diseases are generally regarded as autoimmune diseases arising from the autoimmunity which takes place as an antibody which ought to recognize an exogenous or foreign matter reacts with the autologous tissue or cellular antigen, that is the self-antigen.

It is known that antibodies against self-antigens and self-reactive T cells are found even in healthy persons and that the mere presence of these does not lead to an autoimmune disease. This is because normally a tolerance has been established against self-antigens. In contrast, in patients with autoimmune diseases, the above tolerance to self-antigens is considered to have been disrupted for some reason or other. As the factors involved, those related to antigens, those related to antigen presentation, and those associated with the control of tolerance, among others, may be considered but much remains to be known and the mechanisms involved have not been elucidated as yet.

Meanwhile, growth hormone-releasing factor (GRF; also known as growth hormone-releasing hormone, GHRH) (hereinafter sometimes referred to briefly as GRF) is a 44 residue-peptide hormone secreted from the hypothalamus. This GRF binds to its receptor (growth hormone-releasing factor receptor, GRFR; hereinafter sometimes referred to briefly as GRF receptor) to elevate the intracellular cyclic AMP (hereinafter sometimes referred to briefly as cAMP) concentration through activation of G protein and, hence, promote the release of growth hormone (GH) from the hypothalamus. Moreover, aside from the above pathway, GRF elevates the intracellular cAMP level via a subsidiary pathway to activate protein kinase A (RKA) and increase the influx of calcium ions into the cell to thereby promote release of GH.

GRF is a hormone whose relation to the release (secretion) of GH is well known, and has heretofore been used as a test drug for the diagnosis of diseases of the hypophysis, such as hypophysial dwarf and primary hypothyroidism. However, the relationship of this GRF to autoimmune diseases was not known at all.

### DISCLOSURE OF INVENTION

It is an object of the present invention to provide an anti-autoimmune composition based on a novel mechanism of action. The invention has for its another object to provide a useful method of screening for a candidate substance which may be an active ingredient of said anti-autoimmune composition.

Exploring into the possible influence of growth hormone-releasing factor (GRF) on the onset of experimental autoimmune encephalomyelitis (hereinafter referred to sometimes as EAE), i.e. an animal model of multiple sclerosis which is an autoimmune diseases, the inventor of the present invention discovered that the onset of EAE can be significantly held in check by inhibiting GRF activity.

Specifically, when an attempt was made to experimentally evoke the onset of EAE in artificially GRFR (GRF receptor) gene-defected homo-defective mice (C57BL/6J-Ghrhr^{lit/lit}) (GRFR-homo-defective mice) and hetero-defective mice (C57BL/6J-Ghrhr^{lit/+}) (GRFR-hetero-defective mice), there was found no onset of EAE in the GRFR-homo-defective mice throughout the entire observation period and, even in the GRFR-hetero-defective mice, too, the onset of EAE was significantly suppressed as compared with the wild type mice. Moreover, when a known GRF antagonist for GRF receptor was administered to EAE experimental mice (EAE-induced mice), there was no onset of EAE notwithstanding its experimental induction, indicating that the onset of EAE can be significantly suppressed by the inhibition of GRFR-mediated action of GRF. GRFR-mediated action of GRF.

Based on the above finding that GRF is associated with the onset of autoimmune diseases and that the onset of autoimmune diseases can be suppressed by inhibiting the above activity of GRF, the inventor of the present invention came to be firmly convinced that any substance having GRF-inhibitory activity may be used with advantage as a prophylactic or therapeutic drug for autoimmune diseases. The present invention has been developed on the basis of the above finding.

The present invention is as follows:
1. An anti-autoimmune composition comprising a substance having growth hormone-releasing factor inhibitory activity as an active ingredient.
2. An anti-autoimmune composition according to item 1 wherein the growth hormone-releasing factor inhibitory activity is the action to inhibit the growth hormone-releasing factor receptor-mediated action of growth hormone-releasing factor.
3. An anti-autoimmune composition according to item 2 wherein the growth hormone-releasing factor inhibitory activity is the action to inhibit the binding of growth hormone-releasing factor to growth hormone-releasing factor receptor.
4. An anti-autoimmune composition according to item 3 wherein the substance having growth hormone-releasing factor inhibitory activity is a peptide analog of growth hormone-releasing factor.
5. An anti-autoimmune composition according to item 1 wherein the growth hormone-releasing factor inhibitory activity is the action to inhibit the expression or secretion of growth hormone-releasing factor or the action to inhibit the expression of growth hormone-releasing factor receptor.
6. An anti-autoimmune composition according to any one of items 1 ∼ 5 wherein the autoimmune disease is multiple sclerosis or rheumatoid arthritis.
7. A method of screening for the active ingredient of an anti-autoimmune composition which comprises a step of screening investigational substances for a substance having growth hormone releasing factor inhibitory activity.
8. A screening method according to item 7 characterized in that a screening is made for a substance having growth hormone-releasing factor inhibitory activity with the inhibition of the growth hormone-releasing factor receptor-mediated action of growth hormone-releasing factor being used as a marker.
9. A screening method according to item 8 characterized in that a screening is made for a substance having growth hormone-releasing factor inhibitory activity with the inhibition of the binding of growth hormone-releasing factor to growth hormone-releasing factor receptor being used as a marker.
10. A method of screening for the active ingredient of an anti-autoimmune composition according to item 9 which comprises the following steps (i), (ii) and (iii):
   (i) a step of contacting a growth hormone-releasing factor receptor or a homolog thereof (a growth hormone-releasing factor receptor or equivalent) with a growth hormone-releasing factor or a homolog thereof (a growth hormone-releasing factor or equivalent) in the presence of an investigational substance,
   (ii) a step of measuring the amount of binding of said growth hormone-releasing factor or equivalent to said growth hormone-releasing factor receptor or equivalent and comparing this amount of binding with the amount of binding (control binding) of said growth hormone releasing factor or equivalent to said growth hormone-releasing factor receptor or equivalent as found by contacting said growth hormone-releasing factor receptor or equivalent with said growth hormone-releasing factor or equivalent in the absence of an investigational substance, and
   (iii) a step of selecting an investigational substance showing a reduced amount of binding as compared with the amount of control binding on the basis of the result of the above step (ii).
11. A method of screening for the active ingredient of an anti-autoimmune composition according to item 10 which, in addition to the steps (i), (ii) and (iii) defined in item 10, further comprises:
   (iv) a step of selecting an investigational substance which does not show activity similar to that of growth hormone-releasing factor from among the investigational substances selected in the above step (iii).
12. A screening method according to item 7 characterized in that a screening is made for a substance having growth hormone-releasing factor inhibitory activity with the inhibition of the expression of a growth hormone-releasing factor receptor being used as a marker.
13. A method of screening for the active ingredient of an anti-autoimmune composition according to item 12 which comprises the following steps (i), (ii) and (iii):
   (i) a step of contacting an investigational substance with cells capable of expressing a growth hormone-releasing factor receptor,
   (ii) a step of measuring the amount of expression of the growth hormone-releasing factor receptor in the cells brought into contact with the investigational substance and comparing this amount of expression with the amount of expression (control expression) of the growth hormone-releasing factor receptor in the corresponding control cells not brought into contact with an investigational substance, and
   (iii) a step of selecting an investigational substance showing a reduced amount of expression of growth hormone-releasing factor receptor as compared with the amount of control expression on the basis of the result of the above step (ii).
14. A screening method according to item 7 characterized in that a screening is made for a substance having growth hormone-releasing factor inhibitory activity with the inhibition of the expression or secretion of growth hormone-releasing factor being used as a marker.
15. A method of screening for the active ingredinet of an anti-autoimmune composition according to item 14 which comprises the following steps (i), (ii) and (iii):
   (i) a step of contacting an investigational substance with cells capable of expressing growth hormone-releasing factor,
   (ii) a step of measuring the amount of expression or secretion of growth hormone-releasing factor in said cells brought into contact with the investigational substance and comparing this amount of expression or secretion with the amount of expression (control expression) or secretion (control secretion) of growth hormone-releasing factor in the corresponding control cells not brought into contact with an investigational substance, and
   (iii) a step of selecting an investigational substance showing a reduced amount of expression or secretion of growth hormone-releasing factor as compared with the amount of control expression or control secretion on the basis of the result of the above step (ii).
16. A screening method according to any one of items 7 through 15 wherein the anti-autoimmune composition is a prophylactic or therapeutic drug for multiple sclerosis or rheumatoid arthritis.
17. An anti-autoimmune composition comprising as an active ingredient the substance having growth hormone-releasing factor inhibitory activity obtained by the screening method claimed in any one of items 7 through 16.
18. An anti-autoimmune composition comprising an effective amount of a substance having growth hormone-releasing factor inhibitory activity and a pharmaceutically acceptable carrier.
19. A method for the prophylaxis or therapy of an autoimmune disease which comprises administering an effective amount of an anti-autoimmune composition comprising a substance having growth hormone-releasing factor inhibitory activity and a pharmaceutically acceptable carrier to a subject affected by the autoimmune disease.
20. A method for the prophylaxis or therapy of an autoimmune disease according to item 18 wherein the autoimmune disease is multiple sclerosis or rheumatoid arthritis.
21. Use of a substance having growth hormone-releasing factor inhibitory activity as the active ingredient of an anti-autoimmune composition.
22. Use according to item 21 wherein the anti-autoimmune composition is a prophylactic or therapeutic drug for multiple sclerosis or rheumatoid arthritis.
23. Use of a substance having growth hormone-releasing factor inhibitory activity for the manufacture of an anti-autoimmune composition.
24. Use according to item 23 wherein the anti-autoimmune composition is a prophylactic or therapeutic drug for multiple sclerosis or rheumatoid arthritis.

### BRIEF DESCRIPTION OF THE DRAWINGS

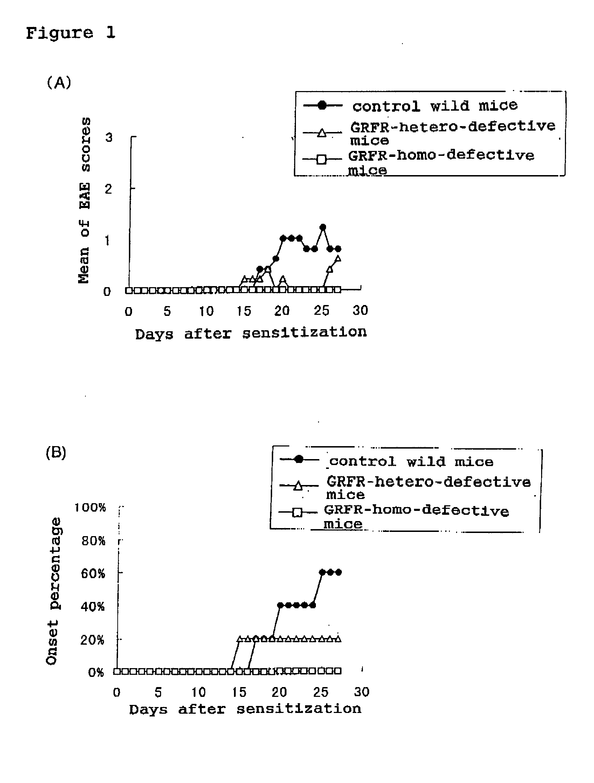

Fig. 1 is a diagrammatic representation of the experimental data generated in Example 1. Specifically, it is a graph showing the onset or non-onset of EAE on artificial induction in GRFR-homo-defective mice (-□-), GRER-hetero-defective mice (-Δ-), and control wild type mice (-•-), respectively, in an exploratory research into the possible involvement of GRF in the onset of EAE.

On the graph, (A) shows the time course of mean EAE score. In wild type mice, the onset of EAE (score 1) began on post-sensitization day 16 and the score became 1.2 on day 25 after sensitization. On the other hand, in GRFR-homo-defective mice, there was not a case of onset throughout the entire observation period (27 days). In GRFR-hetero-defective mice, EAE developed but its mean score was considerably low as compared with the mean score in wild-type mice.

On the graph, (B) represents the time course of EAE onset percentage. In GRFR-homo-defective mice, the onset percentage was zero throughout the entire observation period. In GRFR-hetero-defective mice, the EAE onset percentage was considerably low as compared with wild-type mice.

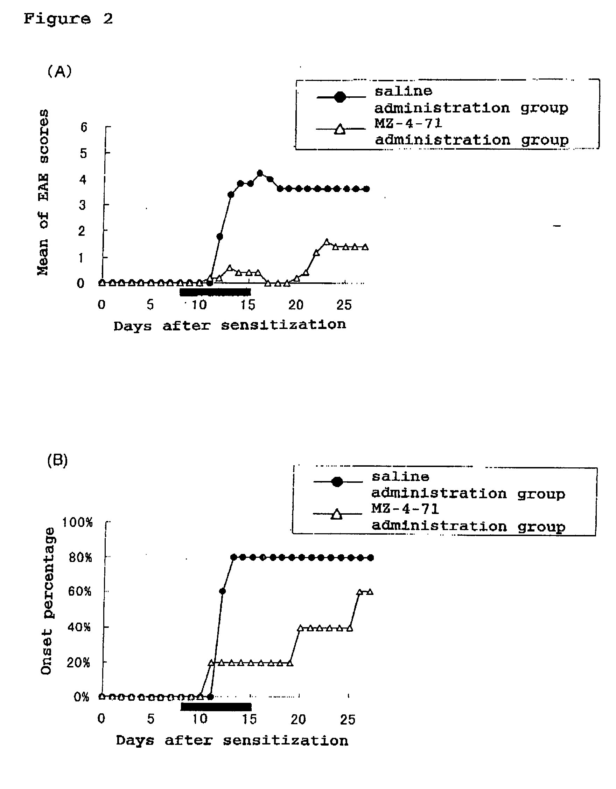

Fig. 2 is a diagrammatic representation of the results of a study in which the onset of EAE was induced in PLSJLFl/J mice by rabbit myelin basic protein (MBP) immunization and pertussis toxin administration and the effect of the GRF antagonist MZ-4-71 on the onset of EAE was investigated.

On the graph, (A) represents the time course of mean EAE score. The time period indicated by a bold black line on the graph indicates the time during which MZ-4-71 or control saline was administered. In the saline administration group, the onset was observed beginning post-sensitization day 12 and the mean score reached 4.2 on post-sensitization day 16. On the other hand, in the MZ-4-71 administration group, the onset was noted beginning post-sensitization day 11 and the mean score reached 0.3 on post-sensitization day 13, which was once followed by the induction of a remission. Then, beginning post-sensitization day 17, remissions were noted in all the mice, with a mean score of 0 registered. Flare-ups occurred beginning post-sensitization day 20 but throughout the subsequent observation period the mean score was never increased beyond the peak of 2.2 (post-sensitization day 23). These results indicate that administration of a GRF antagonist inhibits the onset of EAE.

On the graph, (B) represents the time course of the onset percentage. The time period indicated by a bold black line on the graph corresponds to the time during which MZ-4-71 or control saline was administered. In the saline administration group, precipitating onsets were noted on day 12 to day 13 after sensitization, with the onset percentage reaching 80% on day 13. On the other hand, in the MZ-4-71 administration group, onsets began gradually and the onset percentage was not more than 60% even on post-sensitization day 26. From these results, it is clear that the onset of EAE is inhibited by the administration of a GRF antagonist in terms of onset percentage, too.

### BEST MODE FOR CARRYING OUT THE INVENTION

In this specification, the representation of amino acids, (poly)peptides, (poly)nucleotides, etc. by abbreviations is consistent with the rules of IUPAC-IUB [IUPAC-IUB Communication on Biological Nomenclature, Eur. J. Blochem., 138: 9 (1984)], the Guideline for the Drafting of Specifications or Equivalents Containing Nucleotide Sequences and/or Amino Acid Sequences. (edited by the Japanese Patent Office), and the conventions in the art.

The term "gene" or "DNA" is used in this specification to mean not only a double-stranded DNA but also any of single-stranded DNAs such as the sense strand and antisense strand constituting the same. It is not particularly restricted by length. Therefore, unless otherwise specified, the term "DNA" is used in this specification to mean any and all of the double-stranded DNA inclusive of the human genomic DNA, single-stranded DNA (normal strand) inclusive of cDNA, single-stranded DNA having a sequence complementary to said normal strand (complementary strand), and fragments of these. Furthermore, regardless of the classification of functional domains, the gene or DNA may contain the expression control region, coding region, exons and intron, among others.

As used in this specification, the term "polynucleotide" means whichever of RNA and DNA. The above DNA includes any and all of cDNA, genomic DNA, and synthetic DNA. The above RNA includes any and all of total RNA, mRNA, rRNA, and synthetic RNA.

The term "antibody" is used in this specification to mean a polyclonal antibody, a monoclonal antibody, a chimeric antibody, a single-stranded antibody, and portions of said antibodies retaining an antigen-binding function, such as Fab fragment and fragments derived from a Fab expression library.

### I. Anti-autoimmune composition

As mentioned above, the inventor of the present invention found that the onset of autoimmune diseases can be suppressed or retarded by inhibiting growth hormone-releasing factor (GRF) activity. This finding suggested that any substance capable of inhibiting growth hormone-releasing factor (GRF) activity might be successfully utilized in the prophylaxis or therapy of autoimmune diseases. The anti-autoimmune composition of the present invention, developed on the basis of the above finding, is characterized in that a substance having GRF inhibitory activity is used as an active ingredient.

The "autoimmune diseases" in general are diseases in which the sustained in vivo production of antibodies or lymphocytes which react with the components of the self-tissue causes tissue damages, and can be roughly classified into two groups of diseases, namely the following organ-specific autoimmune diseases and non-organ-specific autoimmune diseases.

Organ-specific autoimmune diseases: Hashimoto's thyroiditis, primary myxedema, thyrotoxicosis, malignant anemia, Good-pasture's syndrome, acute progressive glomerulonephritis, myasthenia gravis, pemphigus vulgaris, bullous pemphigoid, insulin-resistant diabetes, juvenile-onset diabetes, Addison's disease, atrophic gastritis, male sterility, climacterium praecox, lens-induced uveitis (phacoanaphylactic uveitis), multiple sclerosis, ulcerative enteritis, primary biliary cirrhosis, chronic active hepatitis, autoimmune hemolytic anemia, paroxysmal hemoglobinuria, idiopathic thrombocytopenic purpura, and Sjögren's syndrome.

Non-organ-specific autoimmune diseases: rheumatoid arthritis, systemic lupus erythematosus, discoid lupus erythematosus, polymyositis, scleroderma, and mixed connective tissue disease.

The present invention is liberally applicable to such "autoimmune diseases". Preferably, the invention is applied to those autoimmune diseases the onset of which is attributable to a mechanism either identical or similar to that of "experimental autoimmune encephalomyelitis". As such autoimmune diseases, multiple sclerosis, rheumatoid arthritis, and systemic lupus erythematosus can be mentioned by way of example. The more preferred are multiple sclerosis and rheumatoid arthritis, with multiple sclerosis being particularly suited. In this connection, as specific morbid states (symptoms) of rheumatoid arthritis, there can be mentioned collagen-induced arthritis, antigen-induced arthritis, and adjuvant-induced arthritis.

The "anti-autoimmune composition" of the present invention exhibits at least one of the following actions in any of the above-mentioned various autoimmune diseases. i.e. the action to inhibit or suppress its onset, the action to retard its onset, the action to alleviate or ameliorate symptoms after its onset, and the action to cure the disease. In this sense, the "anti-autoimmune composition" of the invention may be called a prophylactic drug for autoimmune diseases, a drug for relieving the symptoms of autoimmune diseases, a drug for achieving a remission of autoimmune diseases, or a therapeutic drug for autoimmune diseases. In particular, the "anti-autoimmune composition" of the invention is outstanding in the action to inhibit or suppress the onset of autoimmune diseases or the action to retard the onset thereof and, as such, can be used with particular advantage as a prophylactic drug for autoimmune diseases.

"Growth hormone-releasing factor: GRF" to which the present invention is addressed is one of hypothalamic hormones which, as described in the "Backgroun Art" section, binds to the GRF receptor to promote the release of growth hormone (GH) from the hypothalamus. In this sense, it is also known as growth hormone-releasing hormone: GHRH.

Its nucleotide sequence and amino acid sequence are known and described in GenBank accession nos; NM_021081 (nucleotide sequence: SEQ ID NO:1), NP_066567 (amino acid sequence: SEQ ID NO:2), NM_031577, NM_010285, AF_242855, U_10156, M_73486, M_ 31658, M_31654 and so forth.

The term "GRF inhibitory activity" in the context of the present invention means broadly the action to inhibit the functional expression of GRF, regardless of the mechanisms of action involved. The "action to inhibit the functional expression of GRF" includes the action to inhibit protein synthesis (inclusive of GRF gene expression) and secretion of GRF and the action to inhibit the GRF receptor-mediated action (signal transduction) of GRF. In this specification, such inhibition of the functional expression of GRF is sometimes referred to merely as inhibition of GRF activity or inhibition of GRF.

The above GRF inhibitory activity can be roughly divided into 1) the action to inhibit the GRF receptor-mediated action (signal transduction) of GRF, 2) the action to inhibit the expression of GRF receptor, and 3) the action to inhibit the expression or secretion of GRF.

The "substance having GRF inhibitory activity" according to the invention need only be a substance having at least one of the above-mentioned inhibitory actions, regardless of its form or nature. Furhtermore, the "substance having GRF inhibitory activity" according to the present invention need only be a substance exerting an inhibitory action at least upon human GRF and it does not particularly matter whether it does or does not inhibit the GRFs derived from mammals other than humans or those derived from other animal species.

The substance having GRF inhibitory activity is now described in detail.

### 1) The substance which inhibits the GRF receptor-mediated action of GRF

As described in the working examples given hereinafter, it has been found that the onset of autoimmune diseases can be inhibited by defecting the GRF receptor and that the onset of autoimmune diseases can be suppressed by causing GRF and a GRF antagonist to compete for the GRF receptor. This finding endorses the proposition that a substance which inhibits the GRF receptor-mediated action of GRF, that is to say a substance which inhibits the GRF receptor-mediated signal transduction of GRF acts in an inhibitory way against antoimmune diseases and, as such, can be the active ingredient of an anti-autoimmune composition.

Here, the substance which inhibits the GRF receptor-mediated action of GRF can be classified according to its site of action, namely (1) the following substance which inhibits the binding of GRF to GRF receptor and (2) the following substance which inhibits the intracellular signal transduction of GRF by acting on the signal transduction system downstream of the GRF receptor.

### (1) The substance which inhibits the binding of GRF to the GRF receptor

This substance inhibits the action (signal transduction) of GRF on GRF receptor by inhibiting the binding of the GRF to the GRF receptor. As specific examples of the substance having such a binding-Inhibitory action, there may be mentioned ① the substance which binds to the GRF receptor to inhibit the binding of GRF to GRF receptor and ② the substance which binds to GRF to inhibit the binding of GRF to GRF receptor. Incidentally the substance ① acts as an antagonist of GRF with respect to the so-called GRF receptor, and it is necessary that even though it binds to the GRF receptor, it should not exhibit an activity (signal transmission) which is qualitatively and quantitatively similar to GRF activity. As used herein, "not exhibit an activity which is similar to GRF activity" includes not only the case where the substance does not have an activity qualitatively similar to GRF activity (deficiency of the function of GRF), but also the case where, although the substance has an activity qualitatively similar to GRF activity, its activity is lower in quantity than GRF activity.

### ① The substance which binds to the GRF receptor to inhibit the binding of GRF to GRF receptor

As specific examples of such substance, there can be mentioned a mutant or modification product of GRF which has a GRF receptor-binding domain (Science, Vol. 218, 585 (1982), Biochem. Biophys. Res. Commun., Vol. 123, 854 (1984)) and is defective of the function of GRF, an anti-GRF receptor antibody, and so forth. In this connection, the substance binding to the GRF receptor need only be a substance having at least a binding affinity for human GRF receptor and it does not particularly matter whether it does or does not inhibit the GRF receptors of mammals other than humans or those of other species of animals.

The above-mentioned mutant or modification product includes but is not limited to a modified GRF protein [or a modified oligo(poly)peptide] having only a GRF receptor-binding domain and a modified GRF protein [or a modified oligo(poly)peptide] defected of the function of GRF by the artificial deletion of an arbitrary number of amino acid residues from the amino acid sequence of the region other than the GRF receptor-binding domain or substitution or insertion (addition) of other amino acids. Furthermore, said modification product of GRF is not particularly restricted in its origin insofar as it binds to at least the human GRF receptor to inhibit the binding of human GRF to the receptor and does not have an activity similar to GRF activity on its own merit as mentioned above.

The amino acid sequence of the binding domain of human GRF for the GRF receptor and the nucleotide sequence encoding the same are presented in the above literature and known. Therefore, the modification product of GRF consisting in the binding domain of human GRF can be easily acquired by one skilled in the art on the basis of the above information.

The technology for artificial modification of the amino acid sequence region other than the GRF receptor-binding domain of GRF is also known to those skilled in the art and such modifications can be made with reference to textbooks such as Molecular Cloning, 2nd Edt., Gold Spring Harbor Laboratory Press (1989). More particularly, modifications can be made by a genetic engineering technique such as site-specific mutagenesis [Methods in Enzymology, 154, 350, 367-382 (1987); ibid 100, 468 (1983); Nucleic Acids Res., 12, 9441 (1984); Zoku Seikagaku Jikken Koza 1, Methods for Gene Research II, ed. by Japanese Biochemical Society, p105 (1986)] etc.; chemical synthesis, e.g. phosphoric triester method or phosphoramidite method [J. Am. Chem. Soc., 89, 4801 (1967); ibid 91, 3350 (1969); Science, 150, 178 (1968); Tetrahedron Lett., 22, 1859 (1981); ibid 24, 245 (1983)]; or a suitable combination of such techniques. Whether the modified GRF protein [a modified oligo(poly)peptide] has lost the function of GRF or not can be determined by administering the modified protein to an animal and measuring the GH secreting ability.

As the anti-GRF receptor antibody as an example of said substance ①, there can be mentioned the antibody to the extracellular domain of GRF receptor [Nature, Vol. 360, p765-768 (1992)]. This anti-GRF receptor antibody need only be one which recognizes and binds at least the extracellular domain of human GRF receptor, and it does not particularly matter whether it is able to recognize and bind the extracellular domains of GRF receptors derived from mammals other than humans or those derived from other species of animals.

The anti-GRF receptor antibody mentioned above can be prepared in the routine manner using a protein having the extracellular domain of human GRF receptor as an immunogen, and may be whichever of a monoclonal antibody and a polyclonal antibody. More particularly, such antibodies can be easily constructed referring to textbooks such as "Antibodies; A Laboratory Manual, Cold Spring Harbor Laboratory Press (1989)", "Current Protocols in Molecular Biology, edit. Ausubel et al. (1987), publish. John Wiley and Sons, Section 11. 12-11.13", etc. Here, the protein having the extracellular domain of human GRF receptor can be easily acquired by one skilled in the art relying on the informaiton about the amino acid sequence of the protein and the nucleotide sequence coding for said amino acid sequence as given in the above-cited literature (Nature).

Furthermore, the substance ① includes other proteins, peptides, and low molecular weight compounds.

Among these. the peptide includes a peptide analog of GRF which binds to human GRF receptor to inhibit the binding of human GRF to the receptor, to mention an example. This peptide analog of GRF by itself does not show an activity similar to GRF activity even upon binding to human GRF receptor to antagonize human GRF, that is to say it is the so-called antagonist of GRF. This peptide analog of GRF need only be one having at least a binding affinity for human GRF receptor and it does not particularly matter whether it is able to bind to GRF receptors of mammals other than humans or those of other species of animals, nor does it particularly matter what the source of the peptide is.

As such peptide analogs of GRF, there can be mentioned MZ-4-71 ([Ibu⁰,D-Arg²,Phe(4-Cl)⁶,Abu¹⁵,Nle²⁷]hGHRH-(1-28)Agm), MZ-4-169 ([Nac⁰,D-Arg²,Phe(4-Cl)⁶,Abu¹⁵,Nle²⁷]hGHRH-(1-29)NH₂), MZ-4-181 ([Nac⁰-His¹,D-Arg²,Phe(4-Cl)⁶,Abu¹⁵,Nle²⁷]hGHRH-(1-29)NH₂) (Proc. Natl. Acad. Sci. USA, Vol.91, p.12298-12302(1994)), MZ-5-156 ([PhAc⁰,D-Arg²,Phe(4-Cl)⁶,Abu¹⁵,Nle²⁷]hGH-RH(1-28)Agm) (Proc. Natl. Acad. Sci.- USA, Vol.96, p.226-231(1999)), JV-1-36 ([PhAc⁰,D-Arg²,Phe(4-Cl)⁶,Arg⁹,Abu¹⁵,Nle²⁷,D-Arg²⁸,Har²⁹]hGH-RH(1-29)NH₂), JV-1-38([PhAc⁰,D-Arg²,Phe(4-Cl)⁶,Har⁹,Tyr(Me)¹⁰,Abu¹⁵,Nle²⁷,D-Arg²⁸,Har²⁹]hGH-RH(1-29)NH₂), JV1-42 ([PhAc⁰-His¹,D-Arg²,Phe(4-Cl)⁶,Arg⁹,Abu¹⁵,Nle²⁷,D-Arg²⁸,Har²⁹]hGH-RH(1-29)NH₂) (Proc. Natl. Acad. Sci. USA, Vol.97, p.1218-1223(2000)), among others. In addition, the peptide analogs described in WO98/16707 (ex. claim 16), and WO91/16923 (ex. claims 1-20) can also be mentioned.

The low molecular weight compound which binds to GRF receptor to inhibit the binding of GRF to GRF receptor can be acquired by applying the screening method of the invention to a known low molecular weight compound library (for example, a low molecular weight compound library of ChemBridge Research Laboratories) or the like.

### ② The substance which binds to GRF to inhibit the binding of GRF to GRF receptor

As a specific example of this substance, the anti-GRF antibody can be mentioned. This anti-GRF antibody includes the antibody against the GRF receptor-binding domain of GRF (Science, Vol. 218, 585 (1982), Biochem. Blophys. Res. Commun., Vol. 123, 854 (1984)). This antibody need only be one which recognizes and binds at least the GRF receptor-binding domain of human GRF and it does not particularly matter whether it is able to bind to GRFs from mammals other than humans or those of other species of animals, nor does it matter what the origin of the production material antigen (protein, polypeptide) is.

This anti-GRF antibody can be prepared in the routine manner using a polypeptide or protein having the GRF receptor-binding domain of human GRF as the immunogen, and may be whichever of a monoclonal antibody and a polyclonal antibody. Specifically, these antibodies can be easily constructed referring to said several textbooks.

The protein having the GRF receptor-binding domain of human GRF can be easily acquired by one skilled in the art relying on the information about the amino acid sequence and nucleotide sequence coding for said amino acid sequence as given in the above-cited literature (Science, Biochem. Biophys. Res. Commun.).

Furthermore, the substance ② includes proteins, peptides, and low molecular weight compounds.

The substance, for example a low molecular compound, which directly binds to GRF to inhibit binding of GRF to GRF receptor can be acquired by applying the screening method of the invention described hereinafter, to a known low molecular weight compound library (for example, a low molecular weight compound library of ChemBridge Research Laboratories) or the like.

### (2) The substance which inhibits the intracellular signal transduction of GRF

As mentioned in the "Background Art" section, GRF as bound to its receptor induces activation of G protein, elevates the cAMP level through this activation, and consequently promotes the release of growth hormone (GH) from the hypothalamus. Moreover, aside from the above pathway, GRF activates protein kinase A through elevation of intracellular cAMP concentration to increase the influx of calcium ions into the cell and promote release of growth hormone.

Therefore, a substance which inhibits the intracellular signal transduction (signal transduction downstream of the GRF receptor) after receptor binding and, as a consequence, inhibits GRF activity is also subsumed in the concept of the GRF-inhibitory substance of the invention.

As an example of such substance, there can be mentioned a substance which induces (elevates) the expression or activities of various factors involved in the intracellular signal transduction of GRF to thereby substantially inhibit said GRF activity and ultimately inhibit the growth hormone release-stimulating action.

More particularly, a protein, peptide or low molecular weight compound which suppresses the expression or activity (activation) of the G protein coupled to GRF receptor can be mentioned as an example.

### 2) The substance which inhibits expression of the GRF receptor

As described in the working examples presented hereinafter, it has been demonstrated that the onset of autoimmune diseases can be suppressed by defecting the GRF receptor. This finding endorses the proposition that a substance which inhibits the expression of GRF receptor functions in an inhibitory way against autoimmune diseases and may therefore be the active ingredient of an anti-autoimmune composition.

The substance which inhibits the expression of GRF receptor need only be one which inhibits the production of a protein having the GRF receptor funciton, regardless of its mechanism of action. For example, there may be mentioned an antisense molecule which arrests the synthesis of GRF receptor (protein) on the nucleic acid level by inhibiting an arbitrary phase, such as replication, transcription or translation, of the GRF receptor gene.

Here, the inhibitory substance in question need only be a substance capable of inhibiting at least the expression of human GRF receptor, and it does not particularly matter whether it inhibits the expression of GRF receptors of non-human mammals or those of other species of animals.

More particularly, there can be mentioned antisense polynucleotides complementary to GRF receptor genes (cf. GenBank accession nos: NM_000823 (SEQ ID NO:3), AY_008835, AY_008834, AF 184896, AB_022597, AB_022596, L_07380, L_07379, etc.).

The antisense molecule mentioned above need not be a polynucleotide having a sequence 100% complementary to the nucleotide sequence of any of said GRF receptor genes but may be a polynucleotide which hybridizes with the nucleotide sequence of the human GRF receptor gene (NM_000823 ,SEQ ID NO:3) under stringent conditions or a mutant thereof [including antisense molecules designed by known techniques, such as a modified poly(oligo)nucleotide, peptide nucleic acid, or the like]. As specific examples, there can be mentioned molecules having a homolgoy of at least 80%, preferably at least 90%, more preferably at least 95% with the complementary sequence to the nucleotide sequence of said GRF receptor gene.

Furthermore, the substance which inhibits the expression of GRF receptor includes proteins, peptides, and low molecular weight compounds.

These proteins, peptides or low molecular weight compounds having an action to inhibit expression of the human GER receptor can be acquired by applying the screening method of the invention, which is described hereinafter, to a known low molecular weight compound library (for example, a low molecular weight compound library of ChemBridge Research Laboratories) or the like.

### 3) The substance which inhibits the expression or secretion of GRF

As described in the working examples presented hereinafter, it has been found that the onset of autoimmune diseases can be controlled or suppressed by defecting the GRF receptor and that the onset of autoimmune diseases can be suppressed by causing GRF and an antagonist of GRF to compete with each other for the GRF receptor. These findings endorse the proposition that a substance which inhibits production of GRF, that is to say a substance which inhibits the expression or secretion of GRF, functions in an inhibitory way against autoimmune diseases and can be the active ingredient of an anti-autoimmune composition.

The substance which inhibits the expression of GRF need only be a substance which inhibits the production of a protein having the GRF function regardless of its mechanism of action. For example, there can be mentioned an antisense molecule having the property to arrest the synthesis of GRF (protein) on the nucleic acid level by inhibiting an arbitrary phase, such as replication, transcription, or translation, of the GRF gene.

Here, the inhibitory substance in question need only be a substance capable of inhibiting at least the expression of human GRF receptor, and it does not particularly matter whether it inhibits the expression of GRF receptors of mammals other than humans or those of other species of animals.

More particularly, there can be mentioned antisense polynucleotides complementary to GRF genes (cf. GenBank accession nos: NM_021081 (SEQ ID NO:1), NM_031577, NM_010285, AF_242855, U_10156, M_73486, M_31658, M_31654 and so forth.)

The antisense molecule mentioned above need not be a polynucleotide having a 100% complementary sequence to the nucleotide sequence of any of said GRF genes but may be a polynucleotide which hybridizes with the nucleotide sequence of a human GRF gene (NM_021081, SEQ ID NO:1) under stringent conditions or a mutant thereof. As specific examples, there can be mentioned molecules having a homolgoy of at least 80%, preferably at least 90%, more preferably at least 95%, with the sequence complementary to the nucleotide sequence of said GRF gene.

Furthermore, the substance which inhibits the expression or secretion of GRF includes proteins, peptides, and low molecular weight compounds. These proteins, peptides and low molecular weight compounds having the property to inhibit the expression or secretion of GER can be acquired by applying the screening method of the invention, which is described hereinafter, to a known low molecular weight compound library (for example, a low molecular weight compound library of ChemBridge Research Laboratories) or the like, respectively.

The above substances having GRF inhibitory activity are all of use as the active ingredient of an anti-autoimmune composition. Moreover, the active ingredient of said anti-autoimmune composition is not particularly restricted to the substances specifically mentioned above but may be one acquired by the screening method of the present invention which is to be described hereinafter.

Therefore, the anti-autoimmune composition to which the present invention is directed includes the composition comprising an effective amount of any of said substances having GRF inhibitory activity and the composition comprising an effective amount of any substance having GRF inhibitory activity as acquired by the screening method described hereinafter. Incidentally, the effective amount of said substance having GRF inhibitory activity need only be a formulating amount of the particular GRF inhibitor substance which enables the anti-autoimmune composition to exhibit the expected GRF-inhibitory effect and is not particularly restricted. Moreover, this anti-autoimmune composition may further contain a pharmaceutically acceptable carrier and various additives in addition to the substance having GRF inhibitory activity.

The kinds or species of said carrier and additives, the morphology of said anti-autoimmune composition, and the administration and dosage of the composition (dosage form, method of administration, and dosage) are described in detail in section III which appears hereinafter.

### II. Method of screening for the active ingredient of an anti-autoimmune composition

As described hereinafter in the working examples, the inventor of the present invention obtained the novel finding that the onset of autoimmune diseases can be suppressed by inhibiting the functional expression of GRF. This finding suggests that a substance having GRF inhibitory activity is of use as the active ingredient of an anti-autoimmune composition. The screening method of the invention which has been developed on the basis of the above finding is designed to acquire such an active ingredient for an anti-autoimmune composition by screening investigational substances for a substance having GRF inhibitory activity.

The "substance having GRF inhibitory activity" which is sought for acquisition by the screening method of the invention need only be a substance which is able to inhibit the functional expression (expression of the functions) of GRF as an ultimate outcome and is not particularly restricted in regard of the mechanism of action involved. The GRF inhibitory activity specifically includes 1) the action to inhibit the GRF receptor-mediated action (signal transduction) of GRF, 2) the action to inhibit the expression of GRF receptor, and 3) the action to inhibit the expression or secretion of GRF. The inhibitory action 1) mentioned above includes (1) the action to inhibit the binding of GRF to GRF receptor and (2) the action to inhibit the intracellular signal transduction of GRF. The substance having GRF inhibitory activity need only be a substance having at least one of said GRF inhibitory actions.

The candidate substance which may be the active ingredient of said anti-autoimmune composition includes nucleic acids, peptides, proteins, organic compounds (inclusive of low molecular weight compounds), inorganic compounds, and so forth. The screening method of the present invention can be applied to substances which can be such candidate substances or samples containing such substances (all of which are collectively referred to as "investigational substance(s)"). The sample containing such a candidate substance (investigational substance) includes cell extracts and expression products of gene libraries, microorganism culture supernatants and bacterial cell components, among others.

The screening method of the invention comprises screening such investigational substances, using the above-mentioned GRF inhibitory actions as markers or indicators, for substances having at least one of said GRF inhibitory actions. The investigational substances so selected are of use as the active ingredient of said anti-autoimmune composition.

The screening method of the invention, as classified by the specific GRF inhibitory action selected as a marker, is now described in detail.

### 1) The screening protocol using the action to inhibit the GRF receptor-mediated action of GRF as a marker

This screening method is a method of screening investigational substances for a substance having GRF inhibitory activity using the inhibition of the GRF receptor-mediated action (signal transduction) of GRF as a marker to acquire an active ingredient for the anti-autoimmune composition.

The "inhibition of the GRF receptor-mediated action of GRF "includes (1) inhibition of the binding of GRF to GRF receptor and (2) Inhibition of the intacellular signal transduotion of GRF.

### (1) A screening protocol using the inhibition of the binding of GRF to GRF receptor as a marker

This screening protocol is a procedure for screening investigational substances for a substance having GRF inhibitory activity using the binding of GRF to GRF receptor as a marker to acquire an active ingredient for the anti-autoimmune composition.

The particulars of this screening protocol are not particularly restricted but can be judiciously designed by one skilled in the art on the basis of the ordinary technical knowledge. However, as an example, there can be mentioned a screening procedure which comprises comparing the binding ability of GRF in the case where GRF is contacted with GRF receptor with the binding ability of GRF in the case where GRF and a investigational substance are contacted with GRF receptor and evaluating the difference. This screening protocol may specifically consist of the following steps (i), (ii) and (iii):
(i) a step of contacting a GRF receptor or a GRF receptor homolog (collectively referred to sometimes as "GRF receptor or equivalent") with GRF or a GRF homolog (collectively referred to sometimes as "GRF or equivalent") in the present of an investigational substance;
(ii) a step of measuring the amount of binding of said GRF or equivalent to said GRF receptor or equivalent in the presence of an investigational substance and comparing this amount of binding with the amount of binding (control binding) of said GRF or equivalent to said GRF receptor or equivalent as found by contacting them in the absence of the investigational substance, and
(iii) a step of selecting the investigational substance which reduces the binding amount compared with the control binding amount based on the result of the above step (ii).

In this connection, the mode of inhibition of the binding of GRF or a GRF homolog (GRF or equivalent) to the GRF receptor or a GRF receptor homolog (GRF receptor or equivalent) is not particularly restricted, i.e. it may be whichever of ① the binding to GRF receptor or equivalent to thereby inhibit the binding of GRF or equivalent to GRF receptor or equivalent and ② the binding to GRF or equivalent to thereby inhibit the binding of GRF or equivalent to GRF receptor or equivalent. In this case, however, particularly in the mode ①, it is necessary to select an investigational substance which does not show an activity similar to GRF activity even upon binding to GRF receptor or equivalent. Therefore, the investigational substance selected by the above screening is preferably further submitted to the following step (iv) of selecting an investigational substance which does not show GRF-like activity from among the investigational substances selected in the above step (iii).

As used herein, "not show an activity similar to GRF activity" include not only the case where the substance does not show an activity qualitatively similar to GRF activity (deficiency of the function of GRF), but also the case where, although the substance has an activity qualitatively similar to GRF activity, its activity is lower in quantity than GRF activity.

The method for the above selection is not particularly restricted but, to mention a specific example, there can be mentioned the method using the in vitro system or in vivo system described in Proc. Natl. Acad. Sci. USA, Vol. 91, p12298-12302 (1994).

The GRF receptor for use in the above screening method of the present invention may be whichever of a native one, a synthetic one, and a recombinant one. Moreover, this GRF receptor is preferably the human GRF receptor but the GRF receptors derived from non-human mammals, typically the mouse, or those derived from other species of animals can also be employed.

In the above screening method, a protein or poly(oligo)peptide having a GRF-binding function (e.g. a GRF receptor mutant (mutein) or derivative having a binding affinity for GRF) may be used in lieu of said GRF receptor. In the present invention, these are referred to collectively as "GRF receptor homolog". For example, said GRF receptor mutant includes those mutants of said known GRF receptors which may be naturally-occurring allels, mutants which do not exist naturally, and mutants having modified amino acid sequences as artificially derived from the amino acid sequences of the parent receptors by partial deletion, substitution, addition or insertion.

Furthermore, the GRF receptor for use in the screening method of the invention need not necessarily has its full-length amino acid sequence but may be one having at least a GRF-binding sequence region, for example the extracellular domain of the GRF receptor. Thus, in the screening method of the invention, a protein or poly(oligo)peptide having at least a GRF-binding region (e.g. the extracellular domain of a GRF receptor) may be used in lieu of said GRF receptor. The "GRF receptor homolog" in the context of the present invention includes such proteins or (poly)peptides. Further, the above-mentioned extracellular domain of the GRF receptor may have been modified, insofar its GRF-binding function is retained, by partial deletion of the amino acid sequence thereof or substitution, addition or insertion of other amino acid residues, and a protein or (poly)peptide having such a modified GRF receptor extracellular domain is also subsumed in the concept of "GRF receptor homolog" according to the present invention.

For the acquisition of a GRF receptor, the following method may be mentioned as an example.

First, based on the amino acid sequence information and the encoding nucleotide sequence information about the GRF receptor of the human origin or the GRF receptor derived from any other species of animals (GenBank accession nos: NM_000823 (nucleotide sequence, SEQ ID NO:3), NP_000814 (amino acid, SEQ ID NO:4), AY_008835, AY_008834, AF_184896, AB_022597, AB_022596, L 07380, L_07379), the probes or PCR primers are constructed in the routine manner and, for example, a cDNA library derived from the human or other creature's pituitary anterior lobe cells is cloned to acquire a cDNA coding for the GRF receptor. Such cloning can be easily carried out by one skilled in the art with reference to a relevant textbook such as Molecular Cloning 2nd Edt., Cold Spring Harbor Laboratory Press (1989).

The GRF receptor cDNA thus prepared is then subcloned into a known expression vector such as pCAGGS (Gene 108, 193-199 (1991)), pcDNA 1.1 or pcDMA 3.1 derivative (both are products of Invitrogen). Thereafter, the resulting recombinant vector is introduced into a suitable host and cultured to construct transformed cells expressing the GRF receptor protein encoded by the transferred GRF receptor cDNA on the cell surface.

The host mentioned above is preferably one of the mammalian cell lines which are generally in broad use as host cells, such as L cells, CHO cells, C127 cells, BHK21 cells, BALB/c3T3 cells (including dihydrofolate reductase-defective, thymidine kinase-defective, and other mutant lines), and COS cells. However, these are not exclusive choices but insect cells, yeast cells, bacterial cells, etc. can also be used. Further, as the method for introduction of said GRF receptor expression vector into host cells, any known method for such introduction can be employed. Thus, for example, the calcium phosphate method (J. Virol., 52, 456-467 (1973)), the method using LT-1 (product of Panvera), and the method using a gene transfer lipid (Lipofectamine, Lipofectin: Gibco-BRL), among others, can be mentioned.

From the transformed cells thus obtained, the GRF receptor is isolated in the routine manner. Thus, for example, in accordance with the method of R. G. Shorr et al. (Prod. Natl. Acad. Sci. USA, 79, 2778-2782 (1982); J. Biol. Chem. 257, 12341-12350 (1982)), the above transformed cells are solubilized using a suitable solubilizing agent (such as β-D-octylglucoside) to give a crude extract containing the GRF receptor. To purify the GRF receptor from this crude extract, any of the methods in routine use in the art, for example the method of J. L. Benovic et al. (Biochem., 23, 4510-4518 (1984)). (e.g. the use of an affinity column carrying an anti-GRF recaptor antibody) can be employed.

A GRF receptor mutant (a mutein of GRF receptor which has an altered amino acid sequence) (a GRF receptor homolog) can be constructed by a genetic engineering technique, such as site-specific mutagenesis [Methods in Enzymology, 154, 350, 367-382 (1987); ibid 100, 468 (1983); Nucleic Acids Res., 12, 9441 (1984); Zoku Seikagaku Jikken Kouza 1, Methods for Gene Research - II, ed. by Japanese Biochemical Society, p105 (1986)] etc.; chemical methods of synthesis, such as phosphoric triester method and phosphoramidite method [J. Am. Chem. Soc., 89, 4801 (1967); ibid 91, 3350 (1969); Science, 150, 178 (1968): Tetrahedron Lett., 22, 1859 (1981); ibid 24, 245 (1983)], or a suitable combination of such techniques, all based on the information about the amino acid sequence of the GRF receptor and the nucleotide sequence coding for the same.

Furthermore, a GRF receptor homolog having only the extracellular domain of the GRF receptor can be prepared by using the cDNA coding for the extraoellular domain of the GRF receptor in lieu of the GRF receptor cDNA in the above procedure for preparation of a GRF receptor and constructing transformed cells in the same manner as above. In this case, the transformed cells can be caused to secrete the objective GRF receptor homolog in the culture supernatant. In this case, the GRF receptor homolog can be isolated and purified from the culture supernatant by using an affinity column supporting the anti-GRF receptor antibody or an antibody against the extracellular domain of the GRF receptor. An alternative method comprises constructing an expression system in such a manner that said extracellular domain will be expressed and produced with a peptide tag added and isolating and purifying the GRF receptor homolog from the culture supernatant of the transformed cells by means of a column supporting a substance having an affinity for said tag.

The nucleotide sequence of the cDNA coding for the "extracellular domain" of the GRF receptor is described in Nature, Vol. 360, 765-768 (1992) as mentioned above, and acquisition of the same can also be easily accomplished by one skilled in the art. Moreover, alteration of the amino acid sequence of the "extracellular domain" can also be carried out by one skilled in the art. The ability or inability of the thus-altered GRF receptor to bind GRF can be assessed by comparing the amount of binding of labeled GRF to the altered GRF receptor, with the amount of binding of labeled GRF to a natural GRF receptor.

The GRF receptor or GRF receptor homolog (GRF receptor or equivalent) obtained by the screening method of the invention may be an isolated or an isolated and purified GRF receptor or equivalent but is not limited thereto. For example, instead of such an isolated GRF receptor or equivalent, cells expressing the GRF receptor or equivalent or a cell membrane fraction containing the GRF receptor or equivalent may be employed.

As said cells expressing the GRF receptor or equivalent, there may be mentioned the transformed cells obtained by introducing a vector harboring said GRF receptor or equivalent cDNA into a suitable host and growing it. These cultured transformant cells have expressed the GRF receptor or equivalent on the cell surface so that these can be directly used in the screening method of the invention.

The cell membrane having the GRF receptor or equivalent can be prepared from said transformed cells. The following method for preparation can be mentioned as an example. First, a hypotonic homogenate buffer (10 mM Tris-HCl buffer, 1 mM EDTA, 0.5 mM PMSF (phenylmethanesulfonyl fluoride) or 1 mM AEBSF, 5 µg/ml aprotinin, 5 µg/ml leupeptin; pH 7.4) is added to the above
cultured transformant cells. The cells are then incubated at 4°C for about 30 minutes for hypotonic disruption, homogenized by
pipetting, and centrifuged (4°C) at 50,000 x g for 30 minutes to give a pellet of the cell membrane fraction. This pellet is
suspended in Tris-HCl buffered saline (Tris-HCl buffer, 154 mM sodium chloride; pH 7.4) to give the objective cell membrane fraction.

As an alternative, such a cell membrane fraction can be obtained from said cultured transformant cells by the method of F. Pietri-Rouxel et al. (Eur. J. Biochem., 247, 1174-1179 (1997)), among other methods.

The GRF for use in the screening method of the invention may be any of native, synthetic, and recombinant ones. Moreover, this GRF is preferably of the human origin but GRFs derived from mammals other than humans, for example the mouse, or those derived from other species of living creatures can likewise be employed. It is also possible to use a protein or poly(oligo)peptide having a binding affinity for the GRF receptor (e.g. a mutant or derivative of GRF capable of binding to the GRF receptor) can be used in lieu of the GRF. In the present invention, these are collectively referred to as "GRF homolog". The above-mentioned mutant of GRF, for instance, includes allels of GRF which are naturally occurring, mutants not existing naturally, and mutants having artificially modified amino acid sequences derived from the amino acid sequence of the GRF by partial deletion, substitution, addition or insertion.

The GRF for use in the screening method of the invention need not have the full-length amino acid sequence of a GRF but may be one having at least the GRF receptor-binding domain. Thus, in the screening method of the invention, a protein or poly(oligo)peptide having at least the GRF receptor-binding domain of a GRF may be used in lieu of the GRF. The "GRF homolog" in the context of the invention includes such proteins or poly(oligo)peptides. Moreover, the above-mentioned GRF receptor-binding domain of a GRF may have an amino acid sequence as modified by partial deletion or by substitution, addition or insertion of other amino acids insofar as the GRF receptor-binding function is still retained, and the protein or poly(oligo)peptide having such a modified GRF receptor-binding domain is also subsumed in the concept of "GRF homolog" according to the present invention.

GRF is basically a protein composed of 44 amino acids. Therefore, it can be synthesized by the routine technology of peptide synthesis on the basis of information on the amino acid sequence of the human GRF or the amino acid sequences of GRFs of other creatures (GenBank accession nos: NP_066567 (SEQ ID NO:2), NM_031577, NM_010285, AF_242855, U_10156, M_73486, M_ 31658,
M_31654). For example, such peptide synthesis can be carried out in accordance with the methods described in the literature [Peptide Synthesis, Interscience, New York, 1966: The Proteins, Vol. 2, Academic Press Inc., New York, 1976; Peptide Gosei (Peptide Synthesis), Maruzen K.K., 1975; Peptide Gosei no Kiso to Jikken (The Fundamentals and Experiments of Peptide Synthesis), Maruzen K.K., 1985; Iyakuhin-no-Kaihatsu - Zoku (Pharmaceutical Development - Continued), Vol. 14: Peptide Gosei (Peptide Synthesis), Hirokawa Shoten, 1991; etc.]. By such methods of peptide synthesis, said GRF homologs can also be easily prepared.

The GRF or GRF homolog can also be constructed by genetic engineering techniques with reference to the information on GRF genes (GenBank accession nos: NM_021081 (SEQ ID NO:1), NM_ 031577, NM_010285, AF_242855, U_10156, M_73486, M_31658, M_ 31654) just as said GRF receptor or GRF receptor homolog. In this case, the cloning of GRF cDNA can be carried out using a human or other creature's GRF-producing tumor cell-derived cDNA library.

Furthermore, GRFs available from commercial sources can also be employed. For example, a GRF can be purchased from Sumitomo Pharmaceutical Co., Ltd.

The GRF or GRF homolog may be used as it is or as labeled with an arbitrary labeling material. The labeling material includes radioisotopes (e.g. ¹²⁵I etc.), fluorescent materials, chemiluminescent materials, biotin, marker proteins or peptide tags, and so forth. As marker proteins, the hitherto-known marker proteins such as alkaline phosphatase (Cell 63, 185-194 (1990)), the Fo fragment of the antibody (GenBank accession number M_87789), and HRP (horse radish peroxidase), among others, can be mentioned. As said peptide tags, the hitherto-known peptide tags such as Myc tag (Glu-Gln-Lys-Lue-Ile-Ser-Glu-Glu-Asp-Ile : SEQ ID NO:5), His tag (His-His-His-His-His-His : SEQ ID NO:6), FLAG tag (Asp-Tyr-Lys-Asp-Asp-Asp-Asp : SEQ ID NO:7), etc. can be mentioned.

Referring to the above step (i) in the screening method (1) of the invention, the conditions under which the GRF receptor or equivalent is brought into contact with the GRF or equivalent in the presence of an investigational substance are not particularly restricted provided that the conditions are conducive to the binding of the GRF receptor or equivalent to the GRF or equivalent in the absence of the investigational substance. In the case where cells expressing a GRF receptor or equivalent or a cell membrane fraction containing a GRF receptor or equivalent is used in lieu of the isolated GRF receptor or equivalent, too, all that is necessary is that the GRF receptor or equivalent of said cells or on the cell membrane thereof may be bound to said GRF or equivalent. Specific examples of such conditions are, in the case of using the cells, ordinary culture conditions under which the cells are viable, and, in the case of using the cell membrane, physiological conditions such as in a Tris-hydrochloric acid buffered saline solution.

The screening for a candidate active ingredient of the anti-autoimmune composition can be carried out by bringing the GRF receptor or equivalent (inclusive of cells expressing the GRF receptor or equivalent or the GRF receptor or equivalent present on the cell membrane thereof) into contact with the GRF or equivalent under the above-mentioned conditions to seek a substance which inhibits their mutual binding. More particularly, an inveatigational substance can be selected as the candidate active ingredient by relying on the finding, as a marker, that the amount of binding between the GRF receptor or equivalent and the GRF or equivalent which takes place when the two are brought into mutual contact in the presence of said investigational substance is decreased as compared with the amount of binding (control binding) found when said GRF receptor or equivalent is brought into contact with said GRF or equivalent in the absence of said investigational substance under otherwise comparable conditions. The above expression "decreased" is meant to include the case in which the binding of the GRF or equivalent to the GRF receptor or equivalent is completely inhibited, that is to say the two are not bound to each other, namely the amount of binding of the GRF or equivalent to the GRF receptor or equivalent is substantially nil.

The amount of binding between the GRF or equivalent and the GRF receptor or equivalent can be measured by a known method, such as a Western blot analysis using an anti-GRF or equivalent antibody. Moreover, when said labeled GRF or equivalent is used as the GRF or equivalent, the GRF or equivalent bound to the GRF receptor or equivalent can be assayed by a method for assay corresponding to the specific labeling material used (e.g. radiometry, fluorometry, etc.).

### (2) A screening protocol using the inhibition of the intercellular signal transduction of GRF as a marker

This screening protocol is a procedure for screening investigational substances for a substance having GRF inhibitory activity using the inhibition of the intercellular signal trans-duction of GRF as a marker to acquire an active ingredient for the anti-autoimmune composition.

This screening protocol may specifically consist of the following steps (i), (ii) and (iii):
(i) a step of contacting a cell expressed a GRF receptor or a GRF receptor homolog (collectively referred to sometimes as "GRF receptor or equivalent") with GRF or a GRF homolog (collectively referred to sometimes as "GRF or equivalent") in the present of an investigational substance;
(ii) a step of measuring the amount of expression or activity of the intercellular signal transduction substance caused by contacting said GRF or equivalent with said cell in the present of an investigational substance, and comparing this amount of expression or activity with the amount of expression or activity corresponding to the above (control amount of expression or activity) obtained by contacting said GRF or equivalent with said cell in the absence of an investigational substance, and
(iii) a step of selecting the investigational substance which varies the amount of expression or activity compared with the control amount of expression or activity based on the result of the above step (ii).

### 2) The screening protocol using the action to inhibit the expression of a GRF receptor as a marker

This screening method is a method of screening investigational substances for a substance having GRF inhibitory activity using the inhibition of the expression of a GRF receptor as a marker to acquire an active ingredient for the anti-autoimmune composition.

The applicable screening method is not particularly limited and it is possible to conduct suitable studies based on common technical knowledge of those skilled in the art. An example that may be cited is a screening method that compares and assesses the amount of expression of a GRF receptor of the applicable cells when allowing and not allowing the investigational substances to come into contact with cells that can express a GRF receptor.

This screening protocol may specifically consist of the following steps (i), (ii) and (iii):
(i) a step of contacting an investigational substance with cells capable of expressing a GRF receptor,
(ii) a step of measuring the amount of expression of the GRF receptor in the cells brought into contact with the investigational substance and comparing this amount of expression with the amount of expression (control amount of expression) of the GRF receptor in the corresponding control cells not brought into contact with an investigational substance, and
(iii) a step of selecting an investigational substance showing a reduced amount of expression of GRF receptor as compared with the amount of control expression on the basis of the result of the above step (ii).

The cells used in this screening method may be any cells that can express GRF receptors, irrespective of the difference between natural and recombinant genes. Moreover, the derivation of the GRF receptors is not particularly limited. The cells may be human derived, or may derive from mammals other than humans such as mice, or from other organisms. Anterior pituitary cells that have genes (DNA) that code a GRF receptor (including cells derived from humans as well as other types of organisms), and initial generation anterior pituitary cultured cells that have been individually prepared may be cited as specific examples of such cells. Moreover, transformed cells that have been adjusted using the previously described method to introduce expression vectors having the cDMA of the GRF receptor may also be used. In addition, tissues that are aggregates of cells are also included in the scope of the cells used in screening.

In step (i) of the aforementioned screening method 2) of the present invention, the conditions for allowing the investigational substance to come into contact with the cells that can express GRF receptors are not particularly limited, but it is preferable to select from among culture conditions (temperature, pH, culture composition, etc.) which will not kill the applicable cells, and in which the genes of the GRF receptors can be expressed.

The selection of candidate substances for the active ingredients in anti-autoimmune composition can be implemented by, for example, allowing the test substance to come in contact with cells that can express a GRF receptor under the aforementioned conditions, and seeking substances that restrict the amount of expression of the GRF receptor. Specifically, the applicable investigational substance can be selected as the candidate substance by using as a marker the fact that the amount of expression of a GRF receptor when culturing cells that can express a GRF receptor in the presence of the investigational substance is lower than the amount of expression of a GRF reoeptor that are obtained when culturing cells that can express a GRF receptor corresponding to the above when not in the presence of the investigational substance (control amount of expression). What "lower than" or "reduced" means here is not only the comparison with the control amount of expression, but also includes when no GRF receptor are expressed at all (when no GRF receptor genes (mRNA) are expressed at all, and when no GRF receptor protein is produced at all). Specifically, this includes substances wherein the amount of expression of a GRF receptor is substantially zero.

In the aforementioned step (ii) of screening method 2), the amount of expression of a GRF receptor can be assessed either by measuring the amount of expression of a GRF receptor gene (mRNA) or by measuring the amount of a GRF receptor protein produced. In addition, the method to measure the amount of a GRF receptor need not be a method to directly measure the amount of expression of gene (mRNA) or the amount of protein produced, but may be any method that reflects these. Specifically, when measuring the amount of expression of a GRF receptor in step (ii), irrespective of any difference between direct and indirect methods, methods that can assess the amount of expression of a GRF receptor gene or the amount of production of a GRF receptor protein may be broadly included.

Specifically, to measure the amount of expression of a GRF receptor (detection and assay), the amount of expression of a GRF receptor mRNA of the cell that can express a GRF receptor may be measured utilizing DNA array or well-known methods such as the Northern blot method, as well as the RT-PCR method that utilizes oligonucleotides having nucleotide sequences complementary to the nucleotide sequence of the applicable GRF receptor mRNA. Moreover, the amount of protein of the a GRF receptor expressed on the surface of the cell that can express a GRF receptor may be measured by implementing such well-known methods as the Western blot method utilizing anti-GRF receptors antibody. The measurement of the amount of expression of GRF receptors (detection and assay) may be implemented by measuring the activity of proteins derived from marker genes, using a cell line into which have been introduced fused genes comprising the marker genes such as reporter genes (e.g., luciferase genes, chloramphenicol- acetyltransferase genes, β-glucuronidase genes, β-galactosidase genes and aequorin genes) linked to the suppression region of the GRF receptor genes [for example, the downstream region of the promoter (Genbank Accession number: AF 267729, AF_121969, AF_127135 of the GRF receptor)]. Screening methods for an active ingredient (target substance) for anti-autoimmune composition of the present invention that index the amount of expression of a GRF receptor include methods that seek the target substance by indexing the amount of expression of related marker genes. Further, the measurement of the production of fused genes and the activity derived from marker genes can be conducted using well-known methods.

### 2) The screening protocol using the action to inhibit the expression or secretion of GRF as a marker

This screening method is a method of screening investigational substances for a substance having GRF inhibitory activity using the inhibition of the expression or secretion of GRF as a marker to acquire an active ingredient for the anti-autoimmune composition.

The applicable screening method is not particularly limited, and may be suitably designed based on common technical knowledge by persons skilled in the art. An example that may be cited is a screening method that compares and assesses the amount of expression or secretion of GRF of the applicable cells when allowing and not allowing the investigational substance to come into contact with cells that can express GRF. This screening protocol may specifically consist of the following steps (i), (ii) and (iii):
(i) a step of contacting an investigational substance with cells capable of expressing GRF,
(ii) a step of measuring the amount of expression or secretion of GRF in said cells brought into contact with the investigational substance and comparing this amount of expression or secretion with the amount of expression (control expression) or secretion (control secretion) of GRF in the corresponding control cells not brought into contact with an investigational substance, and (iii) a step of selecting an investigational substance showing a reduced amount of expression or secretion of GRF as compared with the amount of control expression or control secretion on the basis of the result of the above step (ii).

The cells used in this screening method may be any cells that can express GRF, irrespective of the difference between natural and recombinant genes. Moreover, the derivation of the GRF is not particularly limited. The cells may be human derived, or may derive from mammals other than humans such as mice, or from other organisms. Tumor cells that have genes (DNA) that code GRF (including cells derived from humans as well as other types of organisms), and initial generation tumor cultured cells that have been individually prepared may be cited as specific examples of such cells. Moreover, transformed cells that have been adjusted using the previously described method to introduce expression vectors having the cDNA of the GRF may also be used. In addition, tissues that are aggregates of cells are also included in the scope of the cells used in screening.

In step (i) of the aforementioned screening method 3) of the present invention, the conditions for allowing the investigational substance to come into contact with the cells that can express GRF are not particularly limited, but it is preferable to select from among culture conditions (temperature, pH, culture composition, etc.) which will not kill the applicable cells, and in which GRF genes (mRNA) can be expressed or the GRF proteins produced can be secreted.

The selection of candidate substances for the active ingredients in anti-autoimmune composition can be implemented by, for example, allowing the investigational substance to come in contact with cells that can express GRF under the aforementioned conditions, and seeking substances that restrict the amount of expression or secretion of GRF. Specifically, the applicable test substance can be selected as the candidate substance by using as a marker the fact that the amount of expression or secretion of GRF when culturing cells that can express GRF in the presence of the investigational substance is lower than the amount of expression or secretion of GRF that is obtained when culturing cells that can express GRF corresponding to the above when not in the presence of the investigational substance (control expression or secretion). What "lower than" or "reduced" means here is not only the comparison with the control expression or secretion, but also includes when no GRF is expressed at all (when no GRF genes(mRNA) are expressed at all, and when no GRF protein is produced at all), and when no GRF protein is secreted at all. Specifically, this includes substances wherein the amount of expression or secretion of GRF is substantially zero.

In the aforementioned step (ii) of screening method 3), the amount of expression of GRF can be assessed either by measuring the amount of expression of GRF genes (mRNA) or by measuring the amount of GRF protein produced. Moreover, the amount of GRF secretion can be assessed by measuring the amount of GRF protein included in the culture solution (culture supernatant) obtained by culturing cells that can express GRF. In addition, the method to measure the amount of GRF expression or the amount of GRF secretion need not directly measure the amount of gene (mRNA) expression, the amount of protein production, or the amount of GRF protein in the culture supernatant. Any method that reflects these may be used. Specifically, when measuring the amount of GRF expression or excretion in step (ii), irrespective of the difference between the direct and indirect methods, any method that can assess the amount of GRF genes expressed, or the amount of GRF protein production, or the amount of GRF protein released into the culture supernatant may be broadly included.
To measure the amount of expression of GRF (detection and assay), the amount of expression of GRF mRNA of cells that can express GRF may be measured utilizing DNA array or well-known methods such as the Northern blot method, as well as the RT-PCR method that utilizes oligonucleotides having nucleotide sequences complementary to the nucleotide sequence of the applicable GRF mRNA. Moreover, the amount of GRF production expressed on the surface of the cells that can express GRF may be measured by implementing such well-known methods as the Western blot method utilizing anti-GRF receptor antibody. The measurement of the amount of expression of GRF (detection and assay) may be implemented by measuring the activity of proteins derived from marker genes, using a cell line into which have been introduced fused genes comprising the marker genes, such as reporter genes mentioned above, linked to the suppression region of the GRF genes [for example, the downstream region of the GRF promoter (Genbank Accession number: U_10153)]. Screening methods for an active ingredient (target substance) for anti-autoimmune composition of the present invention that index the amount of expression of GRF include methods that seek the target substance by indexing the amount of expression of marker genes. Further, the measurement of the production of fused genes and the activity derived from marker genes can be conducted using well-known methods.

Moreover, the amount of GRF secretion (detection and assay) can be measured by culturing cells that can express GRF, and by measuring the amount of GRF protein included in the culture supernatant obtained via a well-known method such as, for example, the Western blot method using anti-GRF antibodies.

The candidate substance selected from the test substances by the above screening methods 1)-(1), 1)-(2), 2) or 3) can be obtained as a more practical active ingredient of an anti-autoimmune disease agent, by subjecting the substance to a pharmacological efficacy test using animal models of various human autoimmune diseases, or a safety test.

More particularly, the suppressive effect (prophylactic efficacy, therapeutic efficacy) of the selected candidate substance in multiple sclerosis, for instance, can be evaluated by subjecting the substance to a pharmacological efficacy test using an animal model of multiple sclerosis (for example, EAE-induced mouse). Moreover, the suppressive effect (prophylactic efficacy, therapeutic efficacy) of the selected candidate substance in rheumatoid arthritis can be evaluated by a pharmacological efficacy test using an animal (e.g. mouse) model of collagen-induced arthritis (Trentham DE, Townes AS, Kang AH: Autoimmunity to type II collagens: An experimental model of arthritis J Exp Med 146: 857-868, 1977), antigen-induced arthritis, or adjuvant-induced arthritis (Pearson CM: Development of arthritis, periarthritis and periotitis in rats given adjuvant. Proc Soc Expe Biblo Med 91:95-101, 1956). The substance so selected and acquired can be further subjected to structural analysis and, based on the result, can be produced on a commercial scale by chemical synthesis, biological synthesis (fermentation) or a genetic procedure.

The substance having GRF-inhibitory activity as thus selected by the screening method of the present invention finds application as the active ingredient of the anti-autoimmune composition described hereinbefore. While the autoimmune disease includes the various specific diseases set forth in section (I) of this specification, the GRF inhibitor substance with which the present invention is concerned is particularly efficacious in multiple sclerosis, rheumatoid arthritis and systemic lupus erythematosus, preferably multiple sclerosis and rheumatoid arthritis.

The ''anti-autoimmune composition" of the present invention exhibits at least one of the action to inhibit or suppress the onset of disease, the action to retard the onset of disease, the action to alleviate or relieve symptoms of the disease after onset, and the action to cure the disease in various autoimmune diseases. In this sense, said "anti-autoimmune composition" may be called a prophylactic drug for autoimmune diseases, a drug for alleviating the symptoms of autoimmune diseases, a drug for achieving a remission of autoimmune diseases, or a therapeutic drug for autoimmune diseases, and, therefore, the screening method of the present invention can be a method of screening for active ingredients of such drugs.

The present invention, therefore, provides an anti-autoimmune composition comprising a substance having GRF inhibitory activity as obtainable by the above screening method as an active ingredient.

### III. The mode of use of the anti-autoimmune composition

The dosage form, method of administration, dosage, etc. of the anti-autoimmune composition comprising a substance having GRF inhibitory activity as an active ingredient according to the present invention are now described.

### 1) In the case where the substance having GRF inhibitory activity is in the form of a low molecular weight compound, a peptide or a protein

In the case where the substance having GRF inhibitory activity is in the form of a low molecular weight compound, a peptide or a protein such as an antibody, the substance can be formulated into the ordinary pharmaceutical compositions (pharmaceutical preparations) which are generally used for such forms, and according to the respective forms, such compositions can be administered orally or parenterally. Generally speaking,

the following dosage forms and methods of administration can be utilized.

The dosage form includes such representative forms as solid preparations, e.g. tablets, pills, powders, fine powders, granules, and capsules, and liquid preparations, e.g. solutions, suspensions, emulsions, syrups, and elixirs. These forms can be classified by the route of administration into said oral dosage forms or various parenteral dosage forms such as transnasal preparations, transdermal preparations, rectal preparations (suppositories), sublingual preparations, vaginal preparations, injections (intravenous, intraarterial, intramuscular, subcutaneous, intradermal) and drip injections. The oral preparations, for instance, may for example be tablets, pills, powders, fine powders, granules, capsules, solutions, suspensions, emulsions, syrups, etc. and the rectal and vaginal preparations include tablets, pills, and capsules, among others. The transdermal preparations may not only be liquid preparations, such as lotions, but also be semi-solid preparations, such as creams, ointments, and so forth.

The injections may be made available in such forms as solutions, suspensions and emulsions, and as vehicles, sterilized water, water-propylene glycol, buffer solutions, and saline of 0.4 weight % concentration can be mentioned as examples. These injections, in such liquid forms, may be frozen or lyophilized. The latter products, obtained by lyophilization, are extemporaneously reconstituted with distilled water for injection or the like and administered. The above forms of pharmaceutical composition (pharmaceutical preparation) can be prepared by formulating said substance having GRF inhibitory activity and a pharmaceutically acceptable carrier in the manner established in the art. The pharmaceutically acceptable carrier includes various excipients, diluents, fillers, extenders, binders, disintegrators, wetting agents, lubricants, and dispersants, among others. Other additives which are commonly used in the art can also be formulated. Depending on the form of pharmaceutical composition to be produced, such additives can be judiciously selected from among various stabilizers, fungicides, buffers, thickeners, pH control agents, emulsifiers, suspending agents, antiseptics, flavors, colors, tonicity control or isotonizing agents, chelating agents and surfactants, among others.

The pharmaceutical composition in any of such forms can be administered by a route suited to the objective disease, target organ, and other factors. For example, it may be administered intravenously, intraarterially, subcutaneously, intradermally, or intramuscularly. It may also be directly administered topically into the affected tissue or even orally or rectally.

The dosage and dosing schedule of such a pharmaceutical preparation vary with the dosage form, the disease or its symptoms, and the patient's age and body weight, among other factors, and cannot be stated in general terms. The usual dosage, in terms of the daily amount of the active ingredient for an adult human, may range from about 0.0001 mg to about 500 mg, preferably about 0.001 mg ∼ about 100 mg, and this amount can be administered once a day or in a few divided doses daily.

### 2) In the case where the substance having GRF inhibitory activity is in the form of a polynucleotide

When the substance having GRF inhibitory activity is in the form of a polynucleotide such as the antisense polynucleotide having a nucleotide sequence complementary to the nucleotide sequence of a GRF gene or a GRF receptor gene, the composition may be provided in the form of a drug for gene therapy or a prophylactic drug. Recent years have witnessed a number of reports on the use of various genes, and gene therapy is by now an established technique.

The drug for gene therapy can be prepared by introducing the object polynuoleotide into a vector or transfecting appropriate cells with the vector. The modality of administration to a patient is roughly divided into two modes, viz. the mode applicable to (1) the case in which a non-viral vector is used and the mode applicable to (2) the case in which a viral vector is used. Regarding the case in which a viral vector is used as said vector and the case in which a non-viral vector is used, respectively, both the method of preparing a drug for gene therapy and the method of administration are dealt with in detail in several books relating to experimental protocols [e.g. "Bessatsu Jikken Igaku, Idenshi Chiryo-no-Kosogijutsu (Supplement to Experimental Medicine, Fundamental Techniques of Gene Therapy), Yodosha, 1996; Bessatsu Jikken Igaku: Idenshi Donyu & Hatsugen Kaiseki Jikken-ho (Supplement to Experimental Medicine: Experimental Prtocols for Gene Transfer & Expression Analysis), Yodosha, 1997; Japanese Society for Gene Therapy (ed.): Idenshi Chiryo Kaihatsu Kenkyu Handbook (Research Handbook for Development of Gene Therapies), NTS, 1999, etc.].

The method of preparing a drug for gene therapy and the method of administration are now described in detail for each type of vector used.

### (1) In the case where a non-viral vector is used as the vector

As the non-viral vector, there can be used any desired expression vector capable of causing the gene (polynucleotide) coding for the objective polypeptide or protein to be expressed in vivo, preferably in the human body. Though it is not particularly restricted, pCAGGS [Gene 108, 193-200 (1991)], pBK-CMV, pcDNA 3.1, pZeoSV (Invitrogen, Stratagene), etc. can be mentioned.

Transfer of a polynucleotide into the patient can be achieved by inserting the object polynucleotide into such a non-viral vector (expression vector) in the routine manner and administering the resulting recombinant expression vector. By so doing, the object polynucleotide can be introduced into the patient's cells or tissue.

More particularly, the method of introducing the polynucleotide into cells includes the calcium phosphate transfection (coprecipitation) technique and the DNA (polynucleotide) direct injection method using a glass microtube, among others.

The method of introducing a polynucleotide into a tissue includes the polynucleotide transfer technique using internal type liposomes or electrostatic type liposomes, the HVJ-liposome technique, the modified HVJ-liposome (HVJ-AVE liposome) technique, the receptor-mediated polynucleotide transfer technique, the technique which comprises transferring the polynucleotide along with a vehicle (metal particles) into cells with a particle gun, the naked-DNA direct transfer technique, and the transfer technique using a positively charged polymer, among others.

### (2) In the case where a viral vector is used as the vector

The viral vector includes vectors derived from recombinant adenoviruses and retrovirus. More particularly, there can be mentioned vectors derived from DNA or RNA viruses such as detoxicated retrovirus, adenovirus, adeno-associated virus, herpesvirus, vaccinia virus, poxvirus, poliovirus, sindbis virus, Sendai virus, SV40, human immunodeficiency virus (HIV) and so forth. The adenovirus vector, in particular, is known to be by far higher in infection efficiency than other viral vectors and, from this point of view, the adenovirus vector is preferably used.

Transfer of the polynucleotide into the patient can be achieved by introducing the object polynucleotide into such a viral vector and infecting the desired cells with the recombinant virus obtained. In this manner, the object polynucleotide can be introduced into the cells.

The method of administering the thus-prepared drug for gene therapy to the patient includes the in vivo technique for introducing the drug for gene therapy directly into the body and the ex vivo technique which comprises withdrawing certain cells from a human body, introducing the drug for gene therapy into the cells in vitro, and returning the cells into the human body [Nikkei Science, April, 1994 issue, 20-45; Pharmaceuticals Monthly, 36(1), 23-48, 1994; Supplement to Experimental Medicine, 12(15), 1994; Japanese Society for Gene Therapy (ed.): Research Handbook for Development of Gene Therapies, NTS, 1999]. For use in the prevention or treatment of a systemic autoimmune disease among the autoimmune diseases to which the present invention is addressed, the drug is preferably introduced into the body by the in vivo technique.

When the in vivo method is used, the drug can be administered by a route suited to the object disease, target organ or the like. For example, it can be administered intravenously, intraarterially, subcutaneously or intramuscularly, for instance, or may be directly administered topically into the affected tissue.

The drug for gene therapy can be provided in a variety of pharmaceutical forms according to said routes of administration. In the case of an injectable form, for instance, an injection can be prepared by the per se established procedure, for example by dissolving the active ingredient polynucleotide in a solvent, such as a buffer solution, e.g. PBS, physiological saline, or sterile water, followed by sterilizing through a filter where necessary, and filling the solution into sterile vials. Where necessary, this injection may be supplemented with the ordinary carrier or the like. In the case of liposomes such as HVJ-liposomes, the drug can be provided in various liposome-entrapped preparations in such forms as suspensions, frozen preparations and centrifugally concentrated frozen preparations.

Furthermore, in order that the gene may be easily localized in the neighborhood of the affected site, a sustained-release preparation (e.g. a minipellet) may be prepared and implanted near the affected site or the drug may be administered continuously and gradually to the affected site by means of an osmotic pump or the like.

The polynucleotide content of the drug for gene therapy can be judiciously adjusted according to the disease to be treated, the patient's age and body weight, and other factors but the usual dosage in terms of each polynucleotide is about 0.0001∼ about 100 mg, preferably about 0.001 ∼ about 10 mg. This amount is preferably adminitered several days or a few months apart.

The present invention is further directed to a method for the prophylaxis or therapy of autoimmune diseases which comprises using the anti-autoimmune composition described above. This method can be carried out by administering an effective amount of the anti-autoimmune composition of the invention to a subject affected by an autoimmune disease (an autoimmune disease patient). The autoimmune disease mentioned above includes the various autoimmune diseases mentioned in section (I). The disease is preferably multiple sclerosis, rheumatoid arthritis, or systemic lupus erythematosus, more preferably multiple sclerosis or rheumatoid arthrits.

The subject affected by an autoimmune disease includes not only a subject developing specific symptoms of such an autoimmune disease but also a subject not developing but having a risk for the onset of an autoimmune disease. Therefore, the prophylaxis (prevention) of an autoimmune disease in the context of the present invention includes prevention of the onset of the autoimmmune disease and suppression of the risk level of onset thereof in said subject affected by the autoimmune disease. Moreover, the therapy (treatment) of an autoimmune disease in the context of the present invention includes mitigation or remission of the pathological state (symptoms) of an autoimmune disease in said subject affected by the autoimmune disease.

The dosage form, method of administration, and dosage of the anti-autoimmune disease are as described hereinbefore.

### INDUSTRIAL APPLICABILITY

The present invention provides a new anti-autoimmune composition based on a new action mechanism called GRF inhibition action. Further, the present invention provides a screening method to obtain an active ingredient of an anti-autoimmune composition that manifests an effect based on a new action mechanism called GRF inhibition action. According to the screening method of the present invention, which uses GRF inhibition action as a marker, it is possible to acquire an active ingredient that can prevent and treat the onset of autoimmune disease based on a new action mechanism. Consequently, the screening method of the present invention can be suitably utilized in the development of new drugs that are effective in the prevention or treatment of autoimmune disease.

### EXAMPLES

Specific examples of the present invention will be described below, but the present invention is not limited in any way by these examples.

### Example 1: Study of the affect of missing GRF receptor genes on the onset of experimental autoimmune encephalomyelitis (EAE)

In order to investigate the participation of growth hormone release factor (GRF) in the onset of autoimmune disease, the presence of onset of experimental autoimmune encephalomyelitis (EAE), which is an experimental animal model of human multiple sclerosis, was studied by targeting mice in which the GRFR gene was missing.

The method and results of the experiment are indicated below.

### < Method of Experiment >

Used in the example were artificially GRFR (GRF receptor) gene-defected homo-defective mice (C57BL/6J-Ghrhr^{lit/lit}) (GRFR-homo-defective mice), artificially GRFR gene-defected hetero-defective mice (C57BL/6J-Ghrhr^{lit/+}) (GRFR-hetero-defective mice), and wild mice (C57BL/6J) as the control (all females, and all purchased from The Jackson Laboratory). All the mice were purabased at five weeks of age, and were reared under artificial lighting from 8:00 am to 8:00 pm every day throughout the experiment. The animals were allowed free access to water and solid food (CE-2; Japan CLEA).

The onset of experimental autoimmune encephalomyelitis (EAE) was artificially induced in these mice (five animals per group, total of 15 animals). Specifically, each of these mice was sensitized subcutaneously in the femoral region with 100 µL of an emulsion of a 1:1 mixture of complete Freund's adjuvant (CFA) (manufactured by Difco Co.) to a solution of 2 mg/mL myelin oligodendrocyte glycoprotein (MOG) 35-55 synthetic peptide (amino sequence: Met-Glu-Val-Gly-Trp-Tyr-Arg-Ser-Pro-Phe-ser-Arg-Val-Val-His-Leu-Tyr-Arg-Asn-Gly-Lys, SEQ ID NO:8) (manufactured by Accord Co.) dissolved in physiological saline (manufactured by Otsuka Pharmaceutical Co. Ltd.). Pertussis toxin (manufactured by List Biological Laboratories) (200 ng) dissolved in physiological saline (manufactured by Otsuka Pharmaceutical Co. Ltd.) was intra-abdominally administered immediately after and two days after sensitization. In this manner, experimental autoimmune encephalomyelitis (EAE) was induced.

The body weight of each mouse was measured every day for 27 days, EAE symptoms were observed, and EAE symptom scores were recorded based on the following criteria.

### [EAE symptom scores]

0: No symptoms, 1: Paralysis of tail, 2: Mild paralysis of rear legs, 3: Moderate to severe paralysis of rear legs or mild paralysis of front legs, or both, 4: Complete paralysis of rear legs or moderate to severe paralysis of front legs, or both, 5: Paralysis of all four limbs or struggling with death, 6: Death

### < Results >

The transition of mean values of the EAE symptom scores obtained are exhibited in Fig. 1 (A).

In the results, no onset of experimental autoimmune encephalomyelitis (score 1) was observed throughout the entire observation period (27 days) in the GRFR-homo-defective mice. Moreover, significantly lower EAE scores were observed in the GRFR-hetero-defective mice compared to control wild mice. The time course of the EAE onset percentage is exhibited in Fig. 1 (B).

In the results, the onset percentage in GRFR-homo-defective mice was zero throughout the entire observation period (27 days). The onset percentage of the GRFR-hetero-defective mice remained lower than that of control wild mice.

From these results, it is clear that the onset of EAE is strongly suppressed by the lack of a GRFR gene.

### Example 2: Study of the affect of the administration of GRF antagonist on experimental autoimmune encephalomyelitis (EAE)

In order to investigate the affect of the administration of GRF antagonist on the onset of autoimmune disease, studies were conducted using experimental autoimmune encephalomyelitis (EAE), which is an experimental animal model of human multiple sclerosis.

The method and results of the experiment are indicated below.

### < Method of Experiment >

Wild mice (PLSJLF1/J) (females purchased from The Jackson Laboratory) were used in the experiment. The mice were purchased at five weeks of age, and were reared under artificial lighting from 8:00 am to 8:00 pm every day throughout the experiment. the animals were allowed free access to water and solid food (CE-2; Japan CLEA).
First, these mice (total ten animals) were sensitized by subcutaneous administration in the femoral area of 100 µL of an emulsion in a 1:1 mixture of complete Freund's adjuvant (CFA) (Difco Co.) to 2 mg/mL rabbit myelin basic protein (MBP) (manufactured by Sigma Co.) dissolved in physiological saline (manufactured by Otsuka Pharmaceutical Co, Ltd.). Pertussis toxin (manufactured by List Biological Laboratories) (200 ng) dissolved in physiological saline (manufactured by Otsuka Pharmaceutical Co. Ltd.) was infra-abdominally administered imediately after and two days after sensitization. In this manner, experimental autoimmune encephalomyelitis (EAE) was induced.

Next, GRF antagonist was administered in compliance with the following procedures. The mice (total of ten animals) that had been MBP sensitized and administered pertussis toxin were divided into two groups. One group (five animals) was subcutaneously administered in the dorsal neck region 30 µg of GRF antagonist (MZ-4-71; manufactured by the US company Calbiochem Novabiochem) per mouse once per day from the eighth to 15^{th} day after MBP sensitization (MZ-4-71 administration group). The other group (five animals) was subcutaneously administered in the dorsal neck region an equal amount of physiological saline in the same way as a control once per day from the eighth to 15^{th} day after MBP sensitization (saline administration group). All animals were allowed free access to food and water throughout the entire rearing period including during the aforementioned experimental period.

The body weight of each mouse was measured every day. The EAE symptoms were observed, and EAE symptom scores were recorded based on the following criteria.

### [EAE symptom scores]

0: No symptoms, 1: Paralysis of tail, 2: Mild paralysis of rear legs, 3: Moderate to severe paralysis of rear legs or mild paralysis of front legs, or both, 4: Complete paralysis of rear legs or moderate to severe paralysis of front legs, or both, 5: Paralysis of all four limbs or struggling with death, 6: Death

### < Results >

The results are exhibited in Fig. 2.

The time course of mean values of EAE scores are exhibited in Fig. 2 (A). Compared to the saline administration group. lower EAE scores were observed in the MZ-4-71 administration group. The time course of EAE onset percentage are exhibited in Fig. 2 (B). Compared to the saline administration group, a delay in the beginning of EAE onset was observed in the MZ-4-71 administration group. From these results it is clear that the administration of GRF antagonist suppresses the onset of EAE.

### Example 3: Screening of an active ingredient for an anti-autoimmune composition using GRF receptor-expressed cells

Transformed cells that stably express GRF receptors on the cell surface are acquired by using expression vectors containing GRF receptor cDNA to transform CHO cells and COS cells via well-known methods. Or, the anterior pituitary cells of rats are isolated and prepared using a well-known method (refer to Proc. Natl. Acad. Sci. USA, Vol. 91, p12298-12302 (1994)). Meanwhile, GRF (manufactured by Sumitomo Pharmaceutical Co. Ltd.) is labeled with biotin or a radioisotope (¹²⁵I) using a well-known method.

Next, (i) labeled GRF, or (ii) labeled GRF and the investigational substance are added to the aforementioned GRF receptor-expressed cells (transformed cells, or anterior pituitary cells of rats). After the stipulated time, the bonding of the labeled GRF to the GRF receptor-expressed cells in these reaction series (i) and (ii) are measured, and the two are compared. The investigational substance in which either no bonding is observed in reaction series (ii) or the bonding was low compared to the bonding in reaction series (i), is selected as the candidate substance for an active ingredient of an anti-autoimmune composition.

The candidate substance thus obtained is supplied to a separate screening to detect GRF inhibition action, and to administration tests in EAE-induced animals. By doing this, it ministration tests in EAE-induced animals. By doing this, it is possible to confirm that the aforementioned candidate substance could be an active ingredient for an anti-autoimmune composition.

### Example 4: Screening of an active ingredient for an anti-autoimmune composition using GRF expressible cells

Groups are prepared in which (i) GRF producing tumor cells not added investigational substance, and (ii) GRF producing tumor cells added investigational substance. These groups are cultured for a stipulated period, the cells are then recovered and dissolved by well-known methods, and the amount of mRNA expression of GRF is compared using Northern blot analysis. Or, the cell culture supernatant is collected, and the amount of GRF protein excreted into the culture solution is compared using Western blot analysis. By screening these, if no GRF expression or GRF secretion is observed for group (ii), or if the amount of GRF expression or amount of GRF secretion of group (ii) is less than the results obtained in group (i), then the investigational substance used in (ii) is selected as a candidate substance for an active ingredient of an anti-autoimmune composition.

Moreover, the candidate substance obtained is provided to another screening method to detect GRF inhibition action, and to experiments for administration to EAE-induced animals. By doing this, it is possible to confirm that the aforementioned candidate substance can be an active ingredient for an anti-autoimmune composition.

## Claims

1. An anti-autoimmune composition comprising a substance having growth hormone-releasing factor inhibitory activity as an active ingredient.

2. An anti-autoimmune composition according to Claim 1 wherein the growth hormone-releasing factor inhibitory activity is the action to inhibit the growth hormone-releasing factor receptor-mediated action of growth hormone-releasing factor.

3. An anti-autoimmune composition according to Claim 2 wherein the growth hormone-releasing factor inhibitory activity is the action to inhibit the binding of growth hormone-releasing factor to growth hormone-releasing factor receptor.

4. An anti-autoimmune composition according to Claim 3 wherein the substance having growth hormone-releasing factor inhibitory activity is a peptide analog of growth hormone-releasing factor.

5. An anti-autoimmune composition according to Claim 1 wherein the growth hormone-releasing factor inhibitory activity is the action to inhibit the expression or secretion of growth hormone-releasing factor or the action to inhibit the expression of growth hormone-releasing factor receptor.

6. An anti-autoimmune composition according to any one of Claims 1 - 5 wherein the autoimmune disease is multiple sclerosis or rheumatoid arthritis.

7. A method of screening for the active ingredient of an anti-autoimmune composition which comprises a step of screening investigational substances for a substance having growth hormone releasing factor inhibitory activity.

8. A screening method according to Claim 7 **characterized in that** a screening is made for a substance having growth hormone-releasing factor inhibitory activity with the inhibition of the growth hormone-releasing factor receptor-mediated action of growth hormone-releasing factor being used as a marker.

9. A screening method according to Claim 8 **characterized in that** a screening is made for a substance having growth hormone-releasing factor inhibitory activity with the inhibition of the binding of growth hormone-releasing factor to growth hormone-releasing factor receptor being used as a marker.

10. A method of screening for the active ingredient of an anti-autoimmune composition according to Claim 9 which comprises the following steps (i), (ii) and (iii):
(i) a step of contacting a growth hormone-releasing factor receptor or a homolog thereof (a growth hormone-releasing factor receptor or equivalent) with a growth hormone-releasing factor or a homolog thereof (a growth hormone-releasing factor or equivalent) in the presence of an investigational substance,
(ii) a step of measuring the amount of binding of said growth hormone-releasing factor or equivalent to said growth hormone-releasing factor receptor or equivalent and comparing this amount of binding with the amount of binding (control binding) of said growth hormone releasing factor or equivalent to said growth hormone-releasing factor receptor or equivalent as found by contacting said growth hormone-releasing factor receptor or equivalent with said growth hormone-releasing factor or equivalent in the absence of an investigational substance, and
(iii) a step of selecting an investigational substance showing a reduced amount of binding as compared with the amount of control binding on the basis of the result of the above step (ii).

11. A method of screening for the active ingredient of an anti-autoimmune composition according to Claim 10 which, in addition to the steps (i), (ii) and (iii) defined in Claim 10, further comprises:
(iv) a step of selecting an investigational substance which does not show activity similar to that of growth hormone-releasing factor from among the investigational substances selected in the above step (iii).

12. A screening method according to Claim 7 **characterized in that** a screening is made for a substance having growth hormone-releasing factor inhibitory activity with the inhibition of the expression of a growth hormone-releasing factor receptor being used as a marker.

13. A method of screening for the active ingredient of an anti-autoimmune composition according to Claim 12 which comprises the following steps (i), (ii) and (iii):
(i) a step of contacting an investigational substance with cells capable of expressing a growth hormone-releasing factor receptor,
(ii) a step of measuring the amount of expression of the growth hormone-releasing factor receptor in the cells brought into contact with the investigational substance and comparing this amount of expression with the amount of expression (control expression) of the growth hormone-releasing factor receptor in the corresponding control cells not brought into contact with an investigational substance, and
(iii) a step of selecting an investigational substance showing a reduced amount of expression of growth hormone-releasing factor receptor as compared with the amount of control expression on the basis of the result of the above step (ii).

14. A screening method according to Claim 7 **characterized in that** a screening is made for a substance having growth hormone-releasing factor inhibitory activity with the inhibition of the expression or secretion of growth hormone-releasing factor being used as a marker.

15. A method of screening for the active ingredinet of an anti-autoimmune composition according to Claim 14 which comprises the following steps (i), (ii) and (iii):
(i) a step of contacting an investigational substance with cells capable of expressing growth hormone-releasing factor,
(ii) a step of measuring the amount of expression or secretion of growth hormone-releasing factor in said cells brought into contact with the investigational substance and comparing this amount of expression or secretion with the amount of expression (control expression) or secretion (control secretion) of growth hormone-releasing factor in the corresponding control cells not brought into contact with an investigational substance, and
(iii) a step of selecting an investigational substance showing a reduced amount of expression or secretion of growth hormone-releasing factor as compared with the amount of control expression or control secretion on the basis of the result of the above step (ii).

16. A screening method according to any one of Claims 7 through 15 wherein the anti-autoimmune composition is a prophylactic or therapeutic drug for multiple sclerosis or rheumatoid arthritis.

17. An anti-autoimmune composition comprising as an active ingredient the substance having growth hormone-releasing factor inhibitory activity obtained by the screening method claimed in any one of Claims 7 through 16.

18. An anti-autoimmune composition comprising an effective amount of a substance having growth hormone-releasing factor Inhibitory activity and a pharmaceutically acceptable carrier.

19. Use of a substance having growth hormone-releasing factor inhibitory activity for the manufacture of an anti-autoimmune aomposition.

20. Use according to Claim 19 wherein the anti-autoimmune composition is a prophylactic or therapeutic drug for multiple sclerosis or rheumatoid arthritis.
